# EUROPEAN PATENT SPECIFICATION

(11) **EP 3 968 865 B1**
(45) Date of publication and mention of the grant of the patent: **13.08.2025**
(21) Application number: 20728170.0
(22) Date of filing: 18.05.2020
(51) Int. Cl.: A61B 17/02

(54) **TISSUE SPLAYER**
GEWEBESPREIZER
ÉCARTEUR DE TISSU

(30) Priority: 17.05.2019 GB 201906969
(43) Date of publication of application: 23.03.2022
(73) Proprietor: Cambridge University Hospitals NHS Foundation Trust, Cambridge, Cambridgeshire CB2 0QQ (GB)
(72) Inventor: AGRAWAL, Amit, Cambridge Cambridgeshire CB2 0QQ (GB); KAY, Stuart, Milton Cambridgeshire CB24 6WZ (GB); WILKINS, Rebecca Ann, Cambridge, Cambridgeshire CB25 9AR (GB); GODFREY, Daniel Peterson, Cambridge, Cambridgeshire CB25 9AR (GB); WICKHAM FRENCH, Christopher Paul, Cambridge, Cambridgeshire CB25 9AR (GB)
(74) Representative: Dunleavy, Christopher Squire
(86) International application number: PCT/GB2020/051212
(87) International publication number: WO 2020/234577

(56) References cited:
- US-A- 2 819 521
- US-A- 3 587 591
- US-A- 3 766 910
- US-A- 5 341 798
- US-A- 5 678 579
- US-A1- 2005 027 170
- US-A1- 2005 031 884

## Description

### Field of the invention

The present invention relates to devices for splaying and/or retracting tissues during surgical procedures. In particular, the present invention relates to devices for supporting fingers of a user when splaying and/or retracting tissues during surgical procedures.

### Background

During surgery, following incision, the surgeon and/or other personnel retract the edges of the incision to expose the tissues which the surgeon needs to access. A variety of soft tissue retraction devices have been used, commonly formed from stainless steel and/or other appropriate materials. Typically, conventional retractors will be operated by an assistant whilst a surgeon performs an operation.

Devices intended to provide support for hands whilst playing sports have been described. For example, US 2008/0282445 A1 describes a glove for sports and other uses comprising a palm portion configured to substantially cover a user's palm and palm side surface of the user's fingers and thumb, and a back portion configured to substantially cover the back of a user's hand and back surface of the user's fingers and thumb. One or more inserts are provided between at least two of the fingers, with the inserts webbing the fingers together and spacing them apart. Alternatively, one or more inserts are provided between at least two of the fingers, with the inserts spacing the fingers apart without webbing them together.

Devices for immobilising a hand have been described. For example, US 2006/0276735 A1 describes a low-profile orthotic glove with wrist support attachment for treating loss or impairment of extensor and/or flexor muscle function in the upper extremities, particularly in the wrist, hand and fingers, due to a peripheral neuropathy.

WO 2017/023967 A1 describes a device including a first member connected to a second member via junctions. The members can be statically or pivotally connected at the junctions via arms extending from cross-beams of the members. The junctions can be configured to provide a biasing force. Methods of using the device may include collapsing the device and inserting it through an incision, allowing the biasing force to cause continuous traction and counter-traction adjacent to a leading dissection edge so that retraction of the skin from the tissue can be facilitated during a surgical procedure.

Reference to any patent documents in this background does not constitute an acceptance that such documents belong to a related field of technology to the present application.

US 2005/027170 A1 describes surgical retractors that can prevent their own arms and handle portions from being raised even when a deep portion of a surgical incision needs to be held open. The surgical retractors are provided with a pair of arms which are openable and closable via a hinge portion and include a blade for spreading and holding an incision open at an extremity portion of the arms and a pair of handles for performing an open and close operation of said pair of arms, the handles being provided on said arms respectively. At the extremity portion of each arm is provided a bent arm portion that bends downward in relation to the arm. A bend line L, formed at the extremity portion of said blade provided at the extremity portion of the bent arm portion, is substantially parallel with said arm when seen from a lateral side.

US 5 341 798 A describes the retractor device for human or animal tissues, and more particularly for retracting upper and lower eyelids to expose the underlying eyeball for surgery, comprises a pair of retracting arms each having a spoon articulatingly mounted at one first end of said retracting arm to pivot about an axe that is in substantially perpendicular relationship with said retracting arm. A retracting means joins the second ends of the arms in a cooperating relationship and biases the first ends of said retracting arms into retracted positions. Each spoon has a first curvature adapted to the shape of an eyeball and a second curvature adapted to the shape of an eyelid.

US3587591A describes an obstetrical instrument for rupturing the amniotic sac.

US5678579A describes an apparatus and method for positioning and moving a flexible element in space. The element is formed into a loop mounted on a pair of positioning handles. The loop, which may be a real or a virtual loop, is mounted on the two handles so as to be movable relative to the handles and to form a working segment of the element between corresponding one free end of the handles, each by one hand or both by the fingers (and thumb) of one hand, positions the working segment in space and manipulation of the loop moves the working segment relative to the handles. The apparatus and method find advantageous application in dental flossing devices, a laparoscopic surgical device or cutting or abrading devices, for example.

### Summary

According to a first aspect of the invention, there is provided a tissue splayer including an index finger portion comprising a rigid annulus for receiving an index finger of a user and a middle finger portion comprising a rigid annulus for receiving a middle finger of a user. The tissue splayer also includes a first pivot joint coupling the index finger portion to the middle finger portion to permit the middle finger portion to rotate relative to the index finger portion about a first pivot axis the first pivot joint configured to be located, in use, above a dorsal surface of a user's palm. The tissue splayer also includes a second pivot joint coupling the index finger portion to the middle finger portion to permit the middle finger portion to rotate relative to the index finger portion about the first pivot axis, the second pivot joint configured to be located, in use, below a palmar surface of a user's palm. The tissue splayer also includes a first splay mechanism configured to permit rotation of the middle finger portion away from the index finger portion about the first pivot axis, and to resist rotation of the middle finger portion towards the index finger portion about the first pivot axis.

The splay mechanism may also include a release mechanism configured such that actuation of the release mechanism reduces or removes the resistance of the first splay mechanism to rotation of the middle finger portion towards the index finger portion about the first pivot axis.

The release mechanism may include a button. The release mechanism may include a lever.

The second pivot joint may be substantially co-axial with the first pivot joint.

The tissue splayer may also include a first finger-extension member connected to, received by, or integrally formed with the index finger portion. The tissue splayer may also include a second finger-extension member connected to, received by, or integrally formed with the middle finger portion.

The first finger-extension member may include an annulus for receiving an index finger of a user. The second finger-extension member may include a rigid annulus for receiving a middle finger of a user.

The splay mechanism may include a friction ratchet comprising a torsion spring.

The splay mechanism may include a ratchet comprising a gear and a pawl.

The first splay mechanism may include a gear and two or more pawls configured to engage the gear at increments of rotation angle less than an angular separation between teeth of the gear. The first splay mechanism may contain two or more concentric gears and corresponding pawls. Each concentric gear may be offset from each other concentric gear by an increment of rotation angle less than an angular separation between teeth of the concentric gears.

The tissue splayer may also include a third pivot joint configured to permit rotation about a second pivot axis which is angled relative to the first pivot axis. The tissue splayer may also include a second splay mechanism. The third pivot joint may be connected in series with the first pivot joint to couple the index finger portion to the middle finger portion. The second splay mechanism may be configured to permit rotation of the index finger portion away from the middle finger portion about the second pivot axis, and to resist rotation of the index finger portion towards the middle finger portion about the second pivot axis.

The second pivot axis may be perpendicular to the first pivot axis. The second pivot axis need not intersect the first pivot axis at any point.

The tissue splayer may be configured to be worn over a surgical glove.

The index finger portion and/or the middle finger portion may be attached to, or integrated with, a glove. The glove may be molded as a single piece of a first material. The glove may be configured to be worn over a surgical glove. The glove may be different to a surgical glove.

The index finger portion may encapsulate the index finger of a user when the glove is worn. The middle finger portion may encapsulate the middle finger of a user when the glove is worn. The index finger portion may extend part-way along the index finger of a user when the glove is worn. In other words, the index finger portion may leave the tip of the index finger of a user exposed, for example, as far as a joint between distal and intermediate phalanges. The middle finger portion may extend part-way along the middle finger of a user when the glove is worn. The middle finger portion may leave the tip of the middle finger of a user exposed, for example, as far as a joint between distal and intermediate phalanges.

The glove may omit a ring finger portion. The glove may omit a little finger portion. The glove may omit a thumb portion. The glove may include a ring finger portion. The ring finger portion may encapsulate the ring finger of a user when the glove is worn. The ring finger portion may extend part-way along the ring finger of a user when the glove is worn. The ring finger portion may leave the tip of the ring finger of a user exposed, for example, as far as a joint between distal and intermediate phalanges. The glove may include a little finger portion. The little finger portion may encapsulate the little finger of a user when the glove is worn. The little finger portion may extend part-way along the little finger of a user when the glove is worn. The little finger portion may leave the tip of the little finger of a user exposed, for example, as far as a joint between distal and intermediate phalanges. The glove may include a thumb portion. The thumb portion may encapsulate the thumb of a user when the glove is worn. The thumb portion may extend part-way along the thumb of a user when the glove is worn. The thumb portion may leave the tip of the thumb of a user exposed, for example, as far as a joint between distal and proximal phalanges.

The tissue splayer may be left-handed. The tissue splayer may be right-handed. The tissue splayer may be universal, in other words wearable on either left or right hand. The tissue splayer may be produced in a variety of different sizes.

The tissue splayer may support or include a light source arranged to illuminate a splayed incision. A light source may be attached to, or integrated with, the index finger portion. A light source may be attached to, or integrated with, the middle finger portion. The tissue splayer may support or include a light source attached to, or integrated with, the index finger portion, and a light source attached to, or integrated with, the middle finger portion. The first pivot joint may support or include a light source. The second pivot joint may support or include a light source.

A finger-extension member may be configured for connection to, or reception by, an index finger portion or a middle finger portion of the tissue splayer. The finger-extension member may extend along a longitudinal direction. When received by an index or middle finger portion of the tissue splayer, the longitudinal direction of the finger-extension member may extend along the length of the index or middle finger portion.

The finger-extension member extending along the length of the index or middle finger portion may mean that the finger-extension member extends substantially parallel to the index finger portion or the middle finger portion.

The finger-extension member may include securing means configured to engage one or more portions and/or reinforced portions of the index finger portion or the middle finger portion. The securing means may comprise one or more barbs. The securing means may comprise one or more hooks. The securing means may comprise one or more protrusions extending from a surface of the finger-extension member and perpendicular to the longitudinal direction. The securing means may comprise one or more clips. A cross-section of the finger-extension member may be curved in a plane perpendicular to the longitudinal direction.

The finger-extension member may also include an annulus for receiving a finger of a user.

The finger-extension member may also include, or support, a light source. The finger-extension member may also include, or support, a temperature probe arranged proximate to an end of the finger-extension member which extends away from the tissue splayer. The temperature probe may be arranged so as to measure a temperature of tissue and/or fluids which contact the finger-extension member in use. The finger-extension member may also include, or support, one or more chemical or electrochemical sensors such as, for example, an oxygenation, lactic acid or pH sensor. The finger-extension member may also include, or support, one or more optical sensors. The light source, temperature probe, one or more chemical or electrochemical sensors and/or one or more optical sensors may be housed in an electronics package. The electronics package may be attached to the finger-extension member. The electronics package may be integrated with the finger-extension member.

Finger-extension members having different lengths may be produced and used with the tissue splayer.

A kit may include the tissue splayer and one or more of the finger-extension members.

### Brief Description of the Drawings

Certain embodiments of the present invention will now be described, by way of example, with reference to the accompanying drawings in which:
Figure 1 schematically illustrates a tissue splayer;
Figure 2 schematically illustrates a first tissue splayer;
Figures 3A and 3B schematically illustrate a portion of the first tissue splayer shown in Figure 2;
Figures 4A and 4B schematically illustrate a portion of a second tissue splayer;
Figures 5A and 5B schematically illustrate a portion of a third tissue splayer;
Figures 6A and 6B schematically illustrate a portion of a fourth tissue splayer;
Figure 7 schematically illustrates a portion of a fifth tissue splayer;
Figure 8 schematically illustrates a portion of a sixth tissue splayer;
Figure 9 schematically illustrates a portion of a seventh tissue splayer;
Figure 10 schematically illustrates a portion of an eighth tissue splayer;
Figure 11 schematically illustrates a portion of a ninth tissue splayer;
Figure 12 schematically illustrates a portion of a tenth tissue splayer;
Figure 13 schematically illustrates a portion of an eleventh tissue splayer;
Figures 14A and 14B schematically illustrate a portion of a twelfth tissue splayer;
Figure 15 schematically illustrates force-extension behaviour for an example of the twelfth tissue splayer;
Figure 16 is a schematic projection view of a thirteenth tissue splayer;
Figure 17 is a schematic side-view of the thirteenth tissue splayer shown in Figure 16;
Figure 18 schematically illustrates a portion of a fourteenth tissue splayer;
Figure 19 is a schematic projection view of the thirteenth tissue splayer shown in Figure 16, with a pair of finger-extension members attached;
Figures 20A and 20B schematically illustrate a first configuration for attaching finger-extension members to a tissue splayer;
Figure 21 schematically illustrates a first finger-extension member;
Figure 22 schematically illustrates the first finger-extension member shown in Figure 21 when received by the first configuration shown in Figures 20A and 20B;
Figure 23 schematically illustrates a second finger-extension member;
Figure 24 schematically illustrates the second finger-extension member shown in

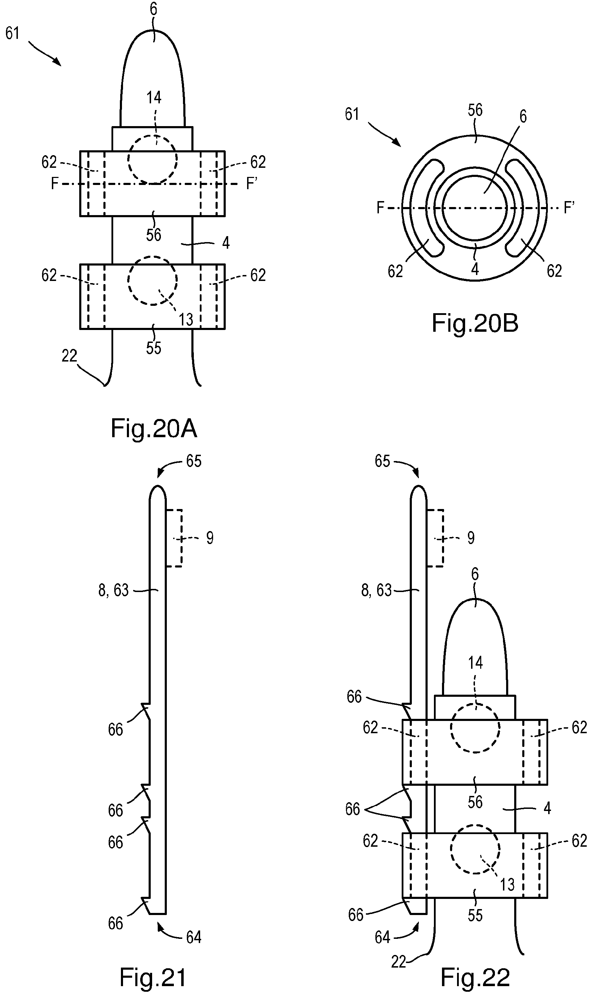

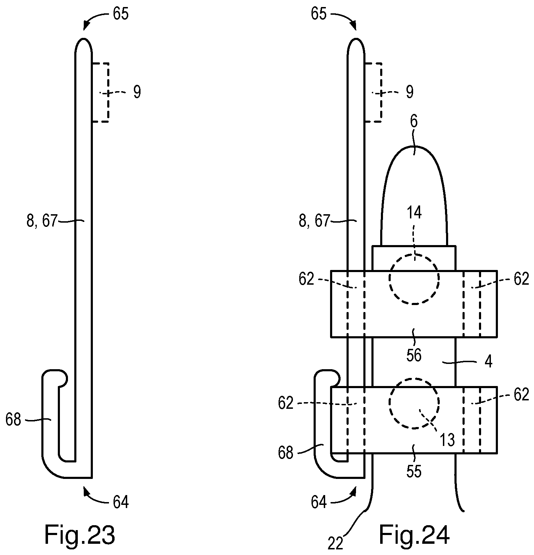

Figure 23 when received by the first configuration shown in Figures 20A and 20B

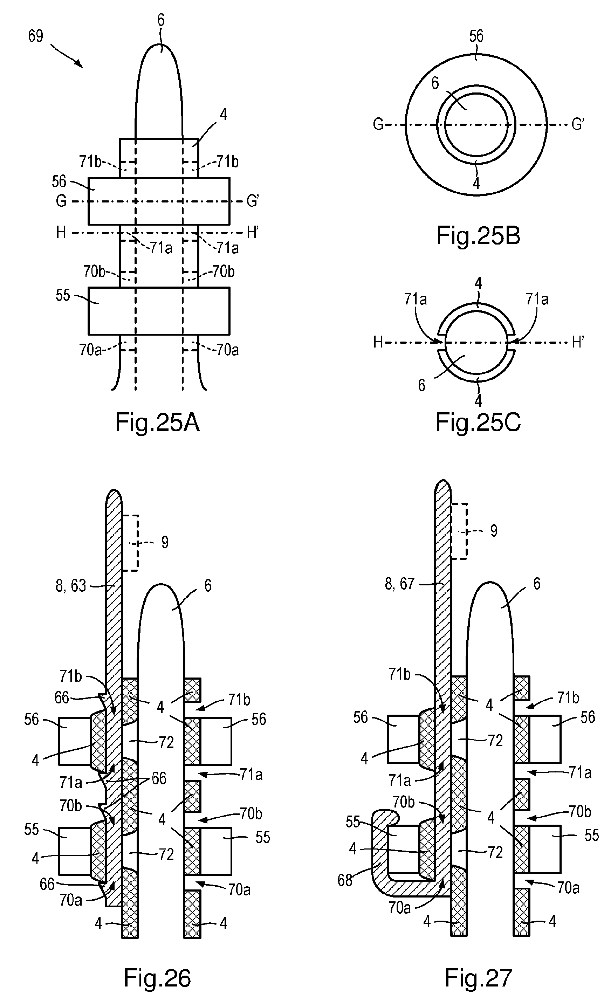

Figures 25A and 25B schematically illustrate a second configuration for attaching finger-extension members to a tissue splayer;
Figure 26 schematically illustrates the first finger-extension member shown in Figure 21 when received by the second configuration shown in Figures 25A and 25B;
Figure 27 schematically illustrates the second finger-extension member shown in Figure 23 when received by the second configuration shown in Figures 25A and 25B;

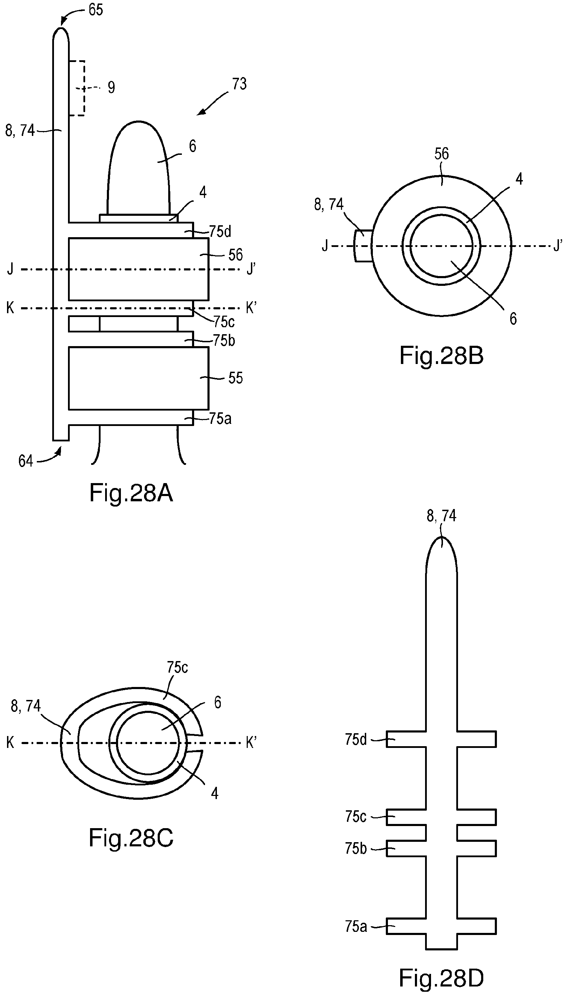

Figures 28A to 28D schematically illustrate a third configuration for attaching a third finger-extension member to a tissue splayer;

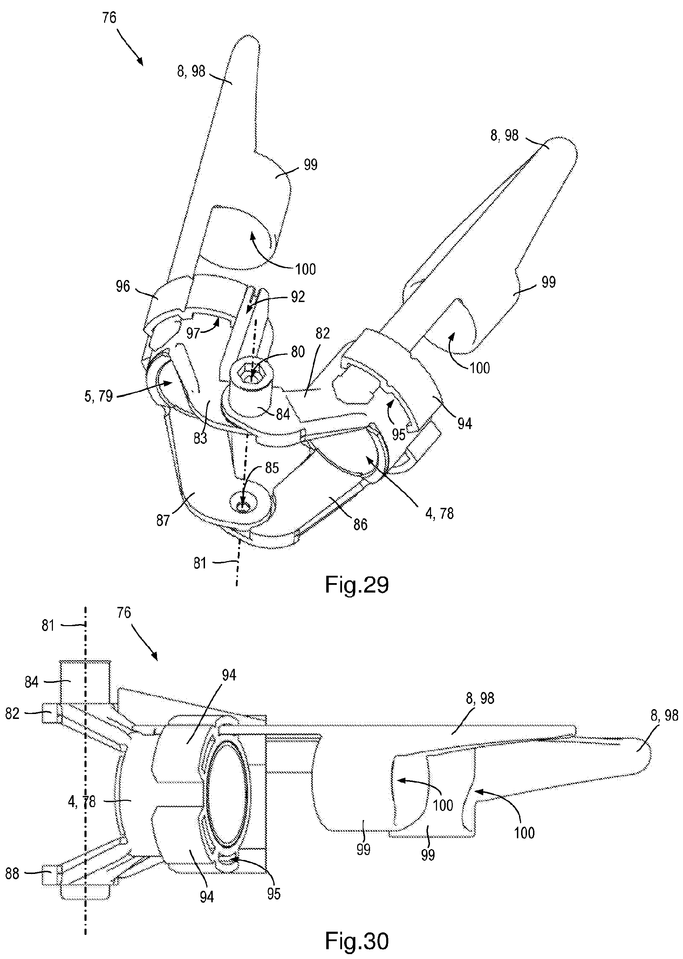

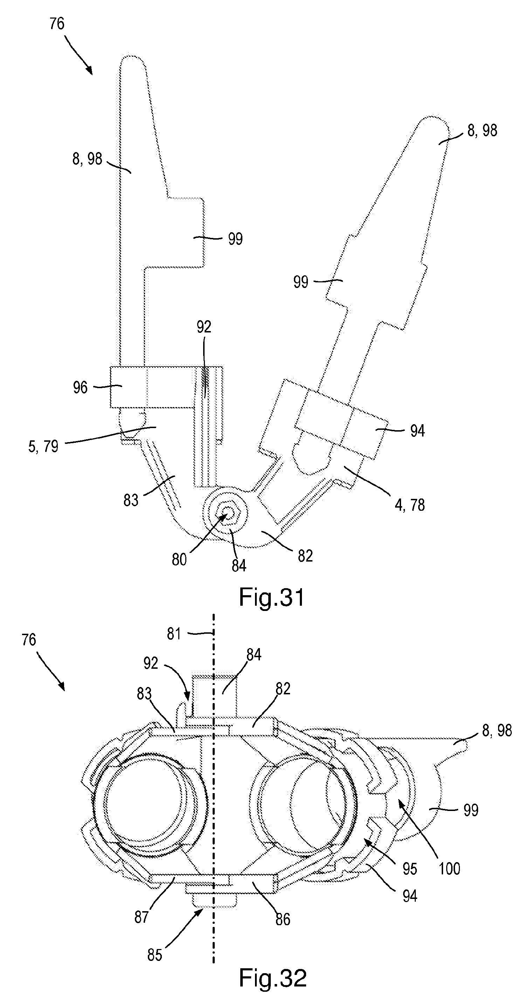

Figures 29 to 32 show projections of a fifteenth tissue splayer from different angles;

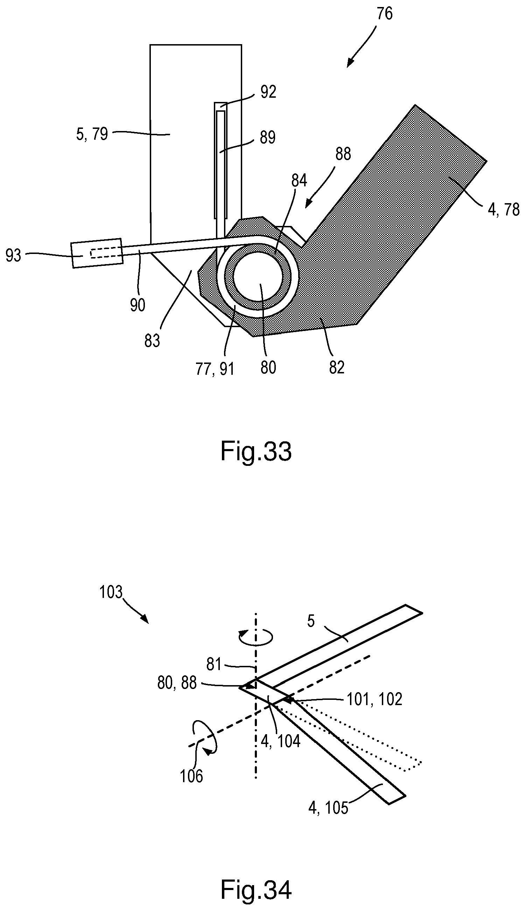

Figure 33 schematically illustrates a first splay mechanism of the fifteenth tissue splayer; and
Figure 34 schematically illustrates a sixteenth tissue splayer.

### Detailed Description of Certain Embodiments

In the following, like parts are denoted by like reference numbers.

Conventional mechanical retractors used in surgical procedures may cause one or more problems during a procedure and/or may cause negative consequences for a patient. Firstly, a lack of feedback to the person operating the retractor may often lead to application of excessive force to the tissues around the edge of an incision, leading to an acute degree of skin deformity and/or tenting. Feedback may include visual feedback, tactile/haptic feedback and so forth. Even if excessive force does not directly cause tearing, the blood supply to surrounding tissues may be reduced or compromised, leading to early or delayed localised cellular necrosis. Excessive force may thus lead to post-surgical complications such as, for example, necrosis of a skin flap, wound dehiscence, infection and so forth. Complications of tissue retraction may have an incidence level in excess of 10%. Patient risk factors are not considered to be modifiable (in either emergent or expedited indication such as in cancer surgery).

A further issue is that the use of conventional retractors tends to narrow (or reduce) the angle of vision for an operating surgeon. The person operating the retractors is typically unable to observe the procedure as a consequence of the general practice of an assistant standing opposite to an operating surgeon to provide counter-traction. When using conventional retractors, touch (tactile/haptic) and/or visual feedback of the effects of the retraction may typically be unavailable to the person operating the conventional retractor.

The current alternative to conventional mechanical retractors is for an operating surgeon to splay their fingers (spread their fingers apart) during the procedure in order to open up the incision. This may provide for a wider visual angle and also provides retraction across the depth to which the surgeon's fingers are inserted (compared to, for example, simply pulling on the skin and surface layers of tissue). Additionally, the natural curvature and deformability of the surgeon's fingers may provide a broader distribution of load on the tissues being splayed compared to often single (or dual) mechanical retractors, which may also apply higher forces using an entire hand and arm. Another potential advantage is that the surgeon's non-dominant fingers will provide the natural and highly-trained mind-eye-hand co-ordination of the surgeon in coordination with dominant fingers being used to perform an operation. By contrast, mechanical retractors may require constant adjustment of passive retraction being supplied. Thus, using the tissue splayer, the time required to perform an operation may be reduced, reducing potential risks associated with longer anaesthesia and/or immobility.

However, whilst providing advantageous outcomes for patients, the finger splaying technique places a significant strain on the surgeon's fingers. A surgeon using this method may experience fatigue and/or pain during a procedure, and in the longer term may be at significant risk of repetitive strain injuries such as, for example, tendinitis, carpal tunnel syndrome and so forth.

The present specification is concerned with a tissue splayer device which may be worn by a surgeon over their gloves, on either the right or left hand, to provide support which enables obtaining the beneficial effects of the finger-splaying technique for the patient and for visibility during the procedure, whilst reducing or removing the negative effects on the surgeon's hand. Importantly, the tissue splayer devices according to the present specification may substantially maintain the dexterity and dynamic control of the supported fingers. This is important, because simply supporting the fingers using static and/or adjustable mechanical supports would not provide the same dynamic control over the applied forces. Dynamic control and dexterity are considered to be contributing factors to the hereinbefore described advantages of the finger-splaying method over conventional mechanical retractors.

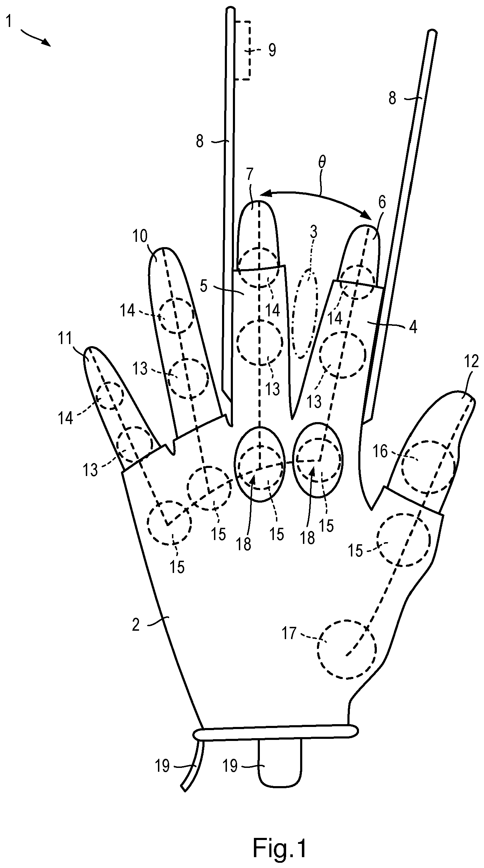

Referring to Figure 1, a tissue splayer 1 useful for understanding the present invention is shown.

The tissue splayer includes a glove 2 and a splay mechanism 3. The glove 2 includes at least an index finger portion 4 and a middle finger portion 5. The splay mechanism 3 is configured to bias an angular separation *θ* of the index and middle finger portions 4, 5 towards a predetermined angular separation (or neutral position) *θₒ* in response to the angular separation *θ* being less than the predetermined angular separation *θₒ*. In other words, when the index and middle finger portions 4, 5 are brought closer together than the predetermined angular separation *θₒ*, the splay mechanism 3 provides a biasing (or restoring) force *F.* The tissue splayer 1 may be left-handed, right-handed or universal (may be worn on either hand). The tissue splayer 1 may be worn over a surgical glove (not shown). The predetermined angular separation *θₒ* may have a value greater than or equal to 10 degrees, and less than or equal to 45 degrees.

In use the index finger 6 is received through the index finger portion 4 and the middle finger 7 is received through the middle finger portion 5. The index and middle finger portions 4, 5 may leave the ends of the fingers 6, 7 uncovered, in order to preserve the fingertip dexterity, touch (tactile) sensation and haptic feedback of the user. Whilst the user may be wearing surgical gloves (not shown) for sterility (and should if the glove 2 of the tissue splayer 1 does not completely enclose the hand), surgical gloves may often be thinner than the walls of the glove 2. This is because the glove 2 has a primarily mechanical function, as opposed to the primarily barrier function of surgical or similar gloves. In some examples, the glove 2 may fully enclose the user's hand, thereby providing barrier functions in addition to mechanical support. In such examples, the walls of the glove 2 may be thinner towards the fingertips, in order to maintain tactile sensations and dexterity of the user.

In some examples, the splay mechanism 3 may also be configured to provide a biasing (or restoring) force *F* in response to the angular separation *θ* being greater than the predetermined angular separation *θₒ*. In other examples, the splay mechanism 3 may provide no, or negligible, biasing force Fin response to the angular separation θ being greater than the predetermined angular separation *θₒ*. In some examples, the splay mechanism 3 may be configured to provide a relatively larger biasing force *F* when the angular separation *θ* is less than the predetermined angular separation *θₒ* compared to when the angular separation *θ* is greater than the predetermined angular separation *θₒ*, i.e. *F*(*θ<θₒ*) *> F*(*θ*)>*θₒ*)*.*

The biasing force *F* provided by the splay mechanism 3 is configured such that a user of the tissue splayer will be capable of displacing the angular separation *θ* to less than the predetermined angular separation *θₒ*. In other words, the splay mechanism 3 does not immobilise the relative movement of the middle and index fingers in use.

In use, a surgeon or other clinical practitioner wearing the tissue splayer 1 may bring their index finger 6 and middle finger 7 together in order to insert the fingers 6, 7 into an incision. The user is able to do this because the biasing force *F* is configured so that it may be overcome by the force of the user's own hand and finger muscles. Once the index and middle fingers 6, 7 are within the incision, the user may relax their muscles, allowing the biasing force F provided by the splay mechanism 3 to push their middle and index fingers 6, 7 apart until equilibrium is reached between the biasing force F and the reaction forces in the tissues being splayed (or spread) apart. Advantageously, the user does not need to constantly apply force using their own intrinsic digital muscles (potentially reducing fatigue). However, the user does have the option to vary the force applied to the tissues around the incision using their own muscle power for short periods in order to expand or contract their field of view. Such dextrous and dynamic control would not be possible using a fixed mechanical retractor or using a conventional fixed and/or adjustable finger spreading device. In this way, the tissue splayer 1 may improve tissue access, improve visualisation/visibility of tissues, retain and/or regain finger dexterity, reduce tissue trauma, reduce surgical complications for the patient, and/or reduce the probability of long term repetitive strain injury to a user.

The function of the tissue splayer 1 is in contrast to, for example, sports gloves intended to help with finger positioning for sports such as golf, basketball and so forth. Sports gloves are often configured so as to effectively immobilise a user's fingers, sometimes with a small amount of mechanical compliance to improve comfort and/or energy absorption. Similarly, orthopaedic finger supports are intended to immobilise one or more fingers of a user, and typically will include no more mechanical compliance than may be necessary for user comfort.

In practical terms, the retention of user dexterity may correspond to the splay mechanism 3 being configured so as to provide a biasing force *F* such that a user of the tissue splayer 1 may displace the angular separation *θ* to at least 5 degrees less than the predetermined angular separation *θₒ*. Preferably, the range of motion of the user's middle and index fingers 6, 7 when wearing the tissue splayer 1 may be larger, for example, the splay mechanism 3 may be configured to provide a biasing force F such that a user of the tissue splayer 1 may displace the angular separation *θ* to at least 10 degrees less than the predetermined angular separation *θₒ*, at least 15 degrees less than the predetermined angular separation *θₒ*, at least 20 degrees less than the predetermined angular separation *θₒ* or at least 25 degrees less than the predetermined angular separation *θₒ*.

The user who should be able to displace the angular separation *θ* less than the predetermined angular separation *θₒ* is a healthy adult. A healthy adult may be a person who is not experiencing any impairment to the joints, tendons, muscles and/or nerves of their hand(s). Any references to a user should be taken as referring to such a healthy adult.

In practice, the function of the splay mechanism 3 to support the index and middle fingers 6, 7 without significantly compromising dexterity and dynamic control may correspond to the biasing force *F* provided by the splay mechanism 3 being less than or equal to 100 N.mm⁻¹ of compression, and optionally the same in tension. The units of N.mm⁻¹ may refer to distances between the proximal interphalangeal joints of a user. In some examples, the biasing force may be lower, for example, the biasing force *F* provided by the splay mechanism 3 may be less than or equal to 50 N.mm⁻¹, or less than or equal to 20 N.mm⁻¹ of compression, and optionally the same in tension.

Since the biasing forces *F* may vary for the same resistance depending on the configuration and positioning of the splay mechanism 3 with respect to a user's fingers, the biasing of the splay mechanism may alternatively be expressed in terms of moments. For example, a moment of less than or equal to 24 Nm may be required to overcome the biasing force *F* and displace the angular separation *θ* to one degree less than the predetermined angular separation *θₒ*. In some examples, a moment of less than or equal to 12 Nm, or less than or equal to 5 Nm may be required in order to overcome the biasing force *F* and displace the angular separation *θ* to one degree less than the predetermined angular separation *θₒ*.

In general, the splay mechanism 3 may be either coupled to the glove 2 or attached to the glove 2. In some examples, the splay mechanism 3 may be integrally formed with the glove 2. For example, the glove 2 may be molded to partially or fully encapsulate the splay mechanism 3.

In some examples, the glove 2 may include only the index finger portion 4 and the middle finger portion. In other examples, the glove 2, or at least those portions covering a user's palm, may be omitted and index and middle finger portions 4, 5 coupled together by a splay mechanism may be received directly over a surgical glove (not shown).

The index finger portion 4 of the tissue splayer 1 may be configured to receive one or more finger-extension members 8. The finger-extension member(s) 8 may be reversibly or irreversibly coupled to the index finger portion 4 using any suitable method such as, for example, clips, hooks, cooperating shapes, barbs, adhesives and so forth. Similarly, the middle finger portion 5 may be configured to receive one or more finger-extension members 8.

The finger-extension member(s) 8 may be useful for further expanding the utility of the tissue splayer 1. For example, finger extension members 8 may enable the finger-splaying technique to be applied to larger incisions than would be possible using the natural spread of a user's index and middle finger-tips.

Finger-extension members 8 may be supplied separately and coupled to/received by the tissue splayer 1 at the time of use. Advantageously, finger-extension members 8 may be provided in a range of sizes, allowing a user to optimise the span of the tissue splayer (i.e. the largest separation) according to the procedure to be performed and the incision size. Alternatively, the tissue splayer 1 may be produced with one or more finger-extension members 8 pre-coupled, or integrally formed with, the index finger portion 4 and/or middle finger portion 5.

When two or more finger-extension members 8 are used, there is no requirement that first and second finger-extension members 8 should be identical. In general, a first finger-extension member 8 and a second, different, finger-extension member 8 may be connected to the same tissue splayer 1, or even to the same finger portion 4, 5.

Although shown in Figure 1 as extending from the sides of the index finger portion 4 and middle finger portion 5, in general finger-extension members 8 may extend from any point around the periphery of the index finger portion 4 and/or middle finger portion 5. For example, finger-extension members 8 may extend in use from any combination of the side surfaces, the dorsal surface and/or the ventral surface of one or both of the index finger portion 4 and the middle finger portion.

In some examples, one or more finger-extension members 8 may optionally include an electronics package 9. When present, the electronics package 9 may be supported by the corresponding finger-extension member 8. Alternatively, the electronics package 9 may be integrally formed within the respective finger-extension member 8.

The electronics package 9 may include one or more of a light source arranged to illuminate an area of tissue being operated upon, a temperature probe so as to measure a temperature of tissue which contacts the finger-extension member 8 in use, one or more chemical or electrochemical sensors configured to measure concentrations of one or more substances in the tissues contacting an end of the finger-extension member 8 (for example an electrochemical oxygenation, lactic acid, or pH sensor). A temperature probe and/or other sensors may be arranged proximate to an end of the finger-extension member 8 which extends away from the tissue splayer 1. The temperature probe and/or other sensors may be arranged on a dorsal surface of a finger-extension member 8 in use. Alternatively, the temperature probe and/or other sensors may be arranged on a palmar (ventral) surface of a finger-extension member 8 in use. In some examples, two or more sensors may be distributed between dorsal and palmar surfaces of a finger-extension member 8 in use. The temperature probe may be advantageous when a surgery uses a thermal tool. For example, an indicator such as a light emitting diode (LED) may indicate local tissue overheating to a user of the tissue splayer 1, who may then cease using a thermal surgical tool to permit the tissues to cool down. The electronics package 9 may include all, or part of, an optical sensor such as a pulse oximeter.

Although Figure 1 illustrates the index finger portion 4 of the glove 2 as extending partway along the length of the index finger 6, in other examples the index finger portion 4 of the glove 2 may encapsulate the index finger 6 of a user when the glove 2 is worn. Similarly, Figure 1 illustrates the middle finger portion 5 of the glove 2 as extending partway along the length of the middle finger 7, in other examples the middle finger portion 5 of the glove 2 may encapsulate the middle finger 7 of a user when the glove 2 is worn.

Figure 1 illustrates a glove 2 which omits a ring finger portion (not shown), a little finger portion (not shown) and a thumb portion (not shown). Consequently, the ring finger 10, little finger 11 and thumb 12 extend from a palm portion of glove 2 via respective holes. In other examples, the glove 2 may include portions extending along one or more of the ring finger 10, little finger 11 and/or thumb 12.

The joints and articulations of the hand have been illustrated in Figure 1 using dashed lines, including the proximal interphalangeal joints 13, the distal interphalangeal joints 14, the metacarpophalangeal joints 15, the interphalangeal joint 16 of the thumb and the intercarpal articulation 17 of the thumb

Optionally, one or two apertures 18 may be provided on the dorsal surface of the glove 2 to overlie one or both of the metacarpophalangeal joints 15 (or knuckles) of the index and middle fingers 6, 7. When the glove 2 is universal (i.e. may be worn on either hand), corresponding pairs of apertures (not shown) may be arranged to lie substantially above and below the metacarpophalangeal joints 15 (or knuckles) of the index and middle fingers 6, 7 in use. The wall thickness of the glove 2 may be thicker than conventional surgical (or similar) gloves, and the inclusion of apertures 18 may help to preserve the movement range of the index and middle fingers 6, 7 about the metacarpophalangeal joints 15.

The glove 2 may optionally include one or more pull tabs 19 which may assist a user when pulling the tissue splayer over their hand or a surgical glove (not shown) worn on their hand.

The glove 2 may be molded as a single piece of a first material such as, for example, an elastomeric material. Suitable elastomeric materials include, but are not limited to, silicone rubber and nitrile rubber. The splay mechanism 3 may be integrally formed with the glove 2. For example, the splay mechanism 3 may be molded along with the glove 2 in a single or multi-shot injection molding process. Alternatively, if the splay mechanism 3 is not suitable for injection molding, the glove 2 may be molded or cast so as to fully or partly enclose the splay mechanism 3. In other examples, the glove 2 and the splay mechanism 3 may be formed separately and subsequently attached, connected, bonded and so forth to form the tissue splayer 1.

In some examples, the glove 2 may include only the index finger portion 4 and the middle finger portion. In other examples, the glove 2, or at least those portions covering a user's palm, may be omitted and index and middle finger portions 4, 5 coupled together by a splay mechanism may be received directly over a surgical glove (not shown).

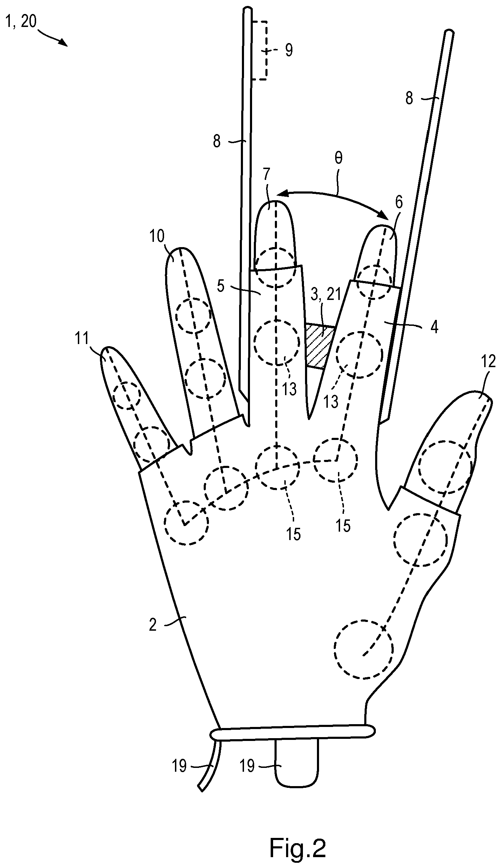

Referring also to Figure 2, a first example 20 of a tissue splayer 1 useful for understanding the present invention is shown (also referred to as the first tissue splayer 20).

The first tissue splayer 20 includes a splay mechanism 3 in the form of a spacer 21 which includes, or is formed of, a resilient material. The spacer 21 is connected between the index and middle finger portions 4, 5. When a user wearing the first tissue splayer 20 changes the angular separation *θ* by displacing their index and middle fingers 6, 7 relative to one another, the spacer 21 acts as a spring and provides a biasing force which acts to bring the index and middle finger portions 4, 5 and the corresponding fingers 6, 7 back to the predetermined angular separation *θₒ* (neutral position).

In the illustration of Figure 2, the spacer 21 connects the index and middle finger portions 4, 5 between positions substantially corresponding to the proximal interphalangeal joints 13 of the middle and index fingers 6, 7. This may be useful because at this position the spacer 21 does not impede independent movements of the intermediate and distal phalanges of the index and middle fingers 6, 7. Some independent motion (dorsal extension and palmar flexion) of the index and middle fingers 6, 7 about the corresponding metacarpophalangeal joints 15 is retained because of the compliance of the material or materials forming the spacer 21. In other words, the index and middle finger portions 4, 5 are not restricted to moving towards (adduction) or away (abduction) from one another, as the splay mechanism 3 in the form of the spacer 21 also permits relative movements of the index and middle fingers 6, 7 of a user in-and-out of the plane of the palm (dorsal extension and palmar flexion).

In some examples, the spacer 21 may be coupled, or attached, to the glove 2. Alternatively, the spacer 21 may be integrally formed with the glove 2. For example, the spacer 21 and the glove 2 may be formed using the first material in a single-shot molding process. Alternatively, the glove 2 and spacer 21 may be formed using two or more materials in a multi-shot molding process.

The previously described functions of the splay mechanism 3 to support the user's index and middle fingers 6, 7 without significantly impeding the dexterity and dynamic control of the fingers 6, 7 may be provided by appropriate configuration of the mechanical compliance of the spacer 21. Mechanical compliance of the spacer 21 may be readily tuned by selection of appropriate materials, cross-sectional area and/or profile of the spacer 21. Preferably, an aspect ratio of the spacer 21 should be such that the spacer 21 does not undergo buckling under compressive loading.

Materials suitable for inclusion in the spacer 21 and/or forming the spacer 21 include compliant materials such as, for example, elastomeric materials, gel materials, foam materials and so forth.

The index finger portion 4 and/or the middle finger portion 5 may be configured to receive finger-extension members 8, or to include finger-extension members 8.

As mentioned hereinbefore, the first tissue splayer 20 may be formed using an injection molding process comprising at least a first material for forming the glove 2. The first material may be elastomeric such as, for example, silicone rubber or nitrile rubber. The first tissue splayer 20 may be formed in a single shot molding, for example the spacer 21 may also be formed from the first material. Alternatively, if the spacer 21 is not formed from the first material, a core portion (not shown) for the spacer 21 may be contained within a mold prior to injection of the first material to form the glove 2 and encapsulate and secure the core portion (not shown) of the spacer 21.

In other examples in which the spacer 21 is formed from different materials to a first material forming the glove 2, the first tissue splayer 20 may be formed in a multi-shot injection molding process. For example, the first material may be used to form the glove 2, whilst one or more further materials may be used to form the spacer 21.

Methods of fabricating the first tissue splayer 20 are not limited to injection molding and derivatives thereof, and any suitable method may be used. Preferably, a tissue splayer 1, for example the first tissue splayer 20, may be fabricated in a sterile environment. Additionally or alternatively, the tissue splayer 1, 20 may be sterilised prior to packing. The tissue splayer 1, 20 should be formed from materials which are capable of being sterilised effectively. The glove 2 of the tissue splayer 1 should preferably be formed from elastomeric materials.

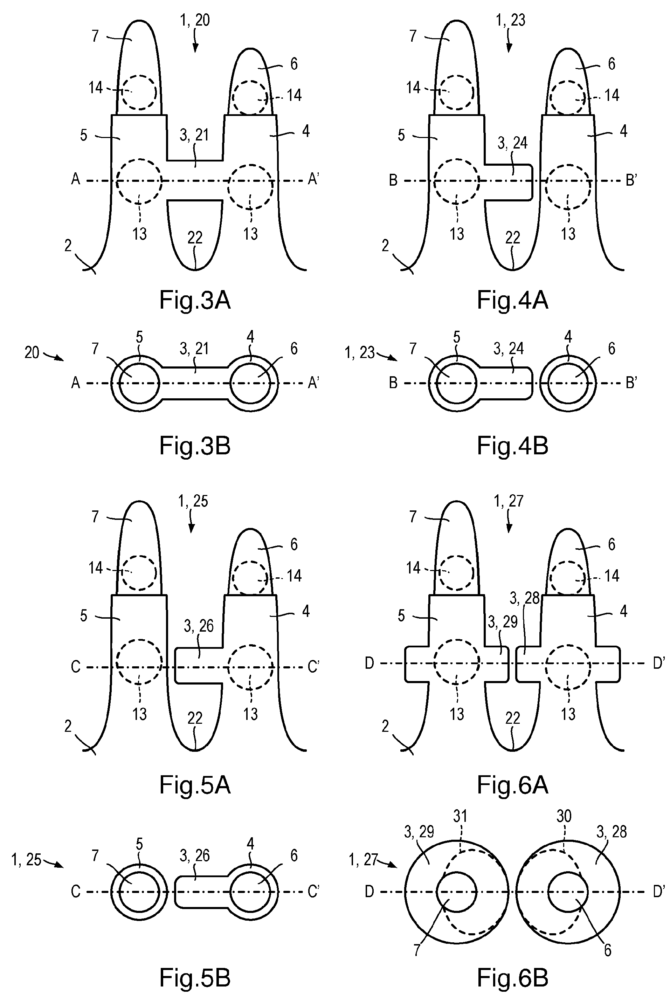

Referring also to Figure 3A the index and middle finger portions 4, 5 of the first tissue splayer 20 are shown. Referring also to Figure 3B, a cross section is shown along the line labelled A-A' in Figure 3A.

The index and middle finger portions 4, 5 are joined to the glove 2 and one another at a connection point 22. The index and middle finger portions 4, 5 receive and partially encapsulate the index and middle fingers 6, 7 respectively in use.

In the first tissue splayer 20, the spacer 21 connects the index finger portion 4 to the middle finger portion 5. Consequently, the spacer of the first tissue splayer 20 applies a biasing force *F* whenever the angular separation *θ* is displaced from the predetermined angular separation *θₒ*, whether in tension or compression. However, in other examples the biasing force *F* may be provided only when the angular separation *θ* is less than the predetermined angular separation *θₒ*, i.e. in compression.

Referring also to Figure 4A, a portion of a second tissue splayer 23 useful for understanding the present invention and including the index and middle finger portions 4, 5 is shown. Referring also to Figure 4B, a cross-section along the line labelled B-B' in Figure 4A is shown.

The second tissue splayer 23 is the same as the first tissue splayer 20, except that the spacer 21 connected between the index and middle finger portions 4, 5 is replaced with a splay mechanism 3 in the form of a second spacer 24 which is only connected to the middle finger portion 5. The second spacer 24 may be attached to, or integrally formed with, the middle finger portion 5. The second spacer 24 extends towards the index finger portion 4, but is not connected to the index finger portion 4. For the second tissue splayer 23, the predetermined angular separation *θₒ* is defined by the neutral (unstressed) length of the second spacer 24. As the index finger portion 4 (and index finger 6) are brought towards the middle finger portion 5 (and middle finger 7), the index finger portion 4 will come into contact with the second spacer 24. Further movement of the index finger portion 4 towards the middle finger portion 5 will compress the second spacer 24, providing a biasing force *F.*

This may permit greater relative mobility for the index and middle fingers 6, 7 of a user than for the first tissue splayer 20, whilst still providing support at angular separations *θ* less than the predetermined angular separation *θₒ*.

The second spacer 24 should have an aspect ratio selected to avoid buckling under compression. Although the second spacer 24 has been illustrated as a rod-like member extending from the middle finger portion 5 towards the index finger portion, the second spacer 24 may alternatively take the form of a thickened ring (or ring portion) extending around (or partially around) a circumference of the middle finger portion 5. In this way, the functioning of the splay mechanism 3 in the form of the second spacer 24 may be provided even when the index and middle fingers 6, 7 are above/below one another with reference to the user's palm (see also Figures 6A and 6B).

Referring also to Figure 5A, a portion of a third tissue splayer 25 useful for understanding the present invention and including the index and middle finger portions 4, 5 is shown. Referring also to Figure 5B, a cross-section along the line labelled C-C' in Figure 5A is shown.

The third tissue splayer 25 is the same as the second tissue splayer 23, except that the second spacer 24 is replaced with a third spacer 26 which is only connected to the index finger portion 4.

Referring also to Figure 6A, a portion of a fourth tissue splayer 27 useful for understanding the present invention and including the index and middle finger portions 4, 5 is shown. Referring also to Figure 6B, a cross-section along the line labelled D-D' in Figure 6A is shown.

The fourth tissue splayer 27 is the same as the first, second or third tissue splayers 20, 23, 25, except that the spacer 21, second spacer 24 or third spacer 26 is replaced by a splay mechanism 3 in the form of a cooperating pair of an index finger spacer 28 and a middle finger spacer 29.

The index finger spacer 28 takes the form of a ring or bulge extending around the circumference of the index finger portion 4. Similarly, the middle finger spacer 29 takes the form of a ring or bulge extending around the circumference of the middle finger portion 5. In this way, regardless of the angle at which index and middle fingers 6, 7 approach one another, the index and middle finger spacers 28, 29 will come into contact, and further movement will generate a biasing force from the compression of the index and middle finger spacers 28, 29.

In this way, the relative motions of the index and middle fingers 6, 7 up, down and away from one another may be almost entirely unimpeded. However, the index and middle finger spacers 28, 29 may provide support for the fingers 6, 7 when used to splay an incision to an angular separation *θ* which is less than a predetermined angular separation *θₒ* defined by the combined thickness of the index and middle finger spacers 28, 29.

In practice, the index and middle finger spacers 28, 29 do not necessarily need to extend all the way around the circumference of the respective finger portions 4, 5. For example, the index finger spacer 28 may have an alternative cross-section profile 30. The alternative cross-section profile 30 maintains a substantially constant radius though a range of angles at which contact with the middle finger spacer 29 is most likely, before dropping to a basic glove 2 thickness for angles from which the middle finger portion 5 cannot approach, for example from opposite to the middle finger 7. Similarly, the middle finger spacer 29 may have an alternative cross-section profile 31.

In the first to fourth tissue splayers 20 23, 25, 27, the spacers 21, 24, 26, 28, 29 have been illustrated as being positioned so as to substantially correspond to the proximal interphalangeal joints 13 of the middle and index fingers 6, 7. However, the spacers 21, 24, 26, 28, 29 may alternatively be disposed corresponding different locations along the length of the index and middle finger portions 4, 5.

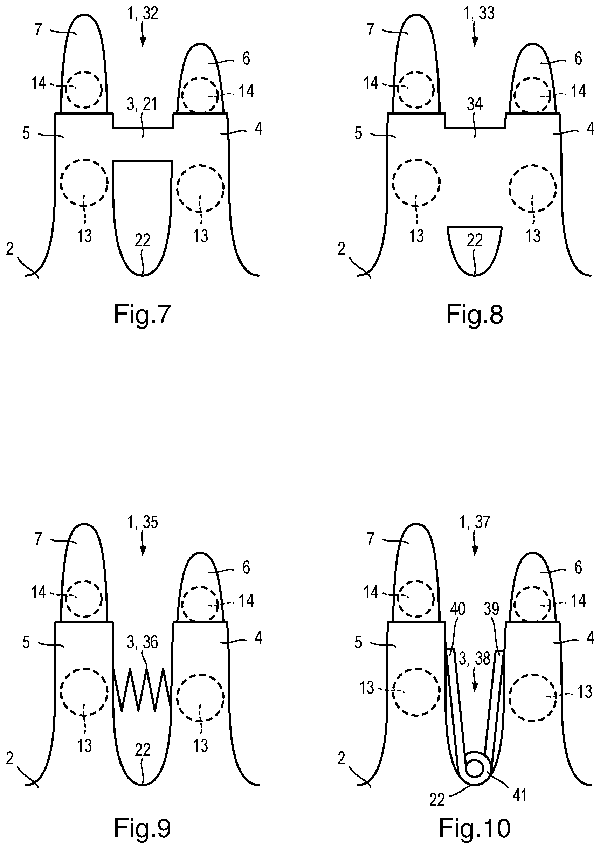

Referring also to Figure 7, a portion of a fifth tissue splayer 32 useful for understanding the present invention and including the index and middle finger portions 4, 5 is shown.

The fifth tissue splayer 32 is the same as the first tissue splayer 20, except that the spacer 21 connects between the index finger portion and the middle finger portion at positions proximate to, without overlapping, the distal interphalangeal joints 14 of a user's index and middle fingers 6, 7 when the fifth tissue splayer 32 is worn.

In other examples, the spacer 21 may connect between the index and middle finger portions 4, 5 at any point between the connection point 22 and the distal interphalangeal joints 14.

The second, third or fourth tissue splayers 23, 25, 27 may be similarly modified so that the respective spacers 24, 26, 28, 29 are disposed at any point between the connection point 22 and the distal interphalangeal joints 14. However, for the fourth tissue splayer 27, the index and middle finger spacers 28, 29 must be arranged at corresponding positions in order to cooperate with one another in use.

The spacers 21, 24, 26, 28, 29 have been illustrated as being relatively narrow along the lengths of the index and/or middle finger portions 4, 5. However, this need not be the case.

For example, referring also to Figure 8, a portion of a sixth tissue splayer 33 useful for understanding the present invention and including the index and middle finger portions 4, 5 is shown.

The sixth tissue splayer 33 is similar to the first or sixth tissue splayers 20, 32, the main difference being that the spacer 21 is replaced by a sixth spacer 34. The sixth spacer 34 substantially spans between the proximal interphalangeal joints 13 of a user's index and middle fingers 6, 7 and a point proximate to, without overlapping, the distal interphalangeal joints 14 of the index and middle fingers 6, 7. In other words, instead of a rod-like member such as the spacer 21, the sixth spacer 34 is more like a webbing connecting the index and middle finger portions 4, 5.

In this way, the support of the index and middle fingers 6, 7 of a user may be supported along a significant fraction of their length, whilst still leaving the distal phalanges free to move independently.

In other examples the sixth spacer 34 may extend all the way down to the connection point 22.

In other examples, the spacers 24, 26, 28, 29 of the second to fourth tissue splayers 23, 25, 27 may be similarly modified to extend for a longer distance along the index and/or middle finger portions 4, 5.

The first to sixth tissue splayers 20, 23, 25, 27, 32, 33 have been described as including splay mechanisms 3 in the form of first to sixth compliant spacers 21, 24, 26, 28, 29, 34. However, the splay mechanism 3 is not limited to compliant spacers 21, 24, 26, 28, 29, 34.

For example, referring also to Figure 9, a portion of a seventh tissue splayer 35 useful for understanding the present invention and including the index and middle finger portions 4, 5 is shown.

The seventh tissue splayer 35 includes a splay mechanism 3 in the form of a compression spring 36, for example a helical compression spring, which connects between the index finger portion 4 and the middle finger portion 5. Similar to the compliant spacers 21, 24, 26, 28, 29, 34 described hereinbefore, the compression spring 36 functions to provide the biasing force *F* when the angular separation *θ* of the index and middle finger portions is displaced from the predetermined angular separation *θₒ*.

In a modification of the seventh tissue splayer 35, the compression spring 36 may be connected only to the index finger portion 4 such that the biasing force *F* is only provided when the angular separation *θ* of the index and middle finger portions is displaced to less than the predetermined angular separation *θₒ*. Alternatively, the compression spring 36 may be connected only to the middle finger portion 5 to obtain similar behaviour.

Referring also to Figure 10, a portion of an eighth tissue splayer 37 useful for understanding the present invention and including the index and middle finger portions 4, 5 is shown.

The eighth tissue splayer 37 includes a splay mechanism 3 in the form of a torsion spring 38 which is arranged between the index finger portion 4 and the middle finger portion 5. Similar to the compliant spacers 21, 24, 26, 28, 29, 34 and compression spring 36 described hereinbefore, the torsion spring 38 functions to provide the biasing force *F* when the angular separation *θ* of the index and middle finger portions is displaced from the predetermined angular separation *θₒ*.

The splay mechanism 3 in the form of the torsion spring 38 includes a first portion 39 extending along the index finger portion 4, a second portion 40 extending along the middle finger portion 5, and a spring portion 41 connecting the first and second portions 39, 40. In the example of the torsion spring 38, the spring portion 41 takes the form of the coil of the torsion spring 38, whilst the first and second portions 39, 40 extend from either end of the coil providing the spring portion 41.

The first and second portions 39, 40 may be attached to the index and/or middle finger portions 4, 5 respectively, using any suitable means. For example, the first and/or second portions 39, 40 may be secured to the index and/or middle finger portions 4, 5 respectively using loops (not shown) formed in the index and/or middle finger portions 4, 5. In other examples, the first portion 39 may be integrally formed with, or encapsulated by, the index finger portion 4 and the second portion 40 may be integrally formed with, or encapsulated by, the middle finger portion 5. For example, the index and middle finger portions 4, 5, along with the rest of the glove 2, may be molded around the splay mechanism 3 in the form of a torsion spring 38. In other example, the torsion spring 38 may be connected to, or integrally formed with, only one of the index and middle finger portions 4, 5. In such examples, the angular separation *θ* may be displaced to more than the predetermined angular separation *θₒ* without the torsion spring 38 providing a biasing (restoring) force *F.*

Although the first and second portion 39, 40 have been illustrated in Figure 10 as extending from the spring portion 41 as straight lines, in practice either of both of the first and second portions 39, 40 may be curved to conform to the interior surfaces of the index and/or middle finger portions 4, 5, or the index and/or middle fingers 6, 7.

In some examples, the first and second portions 39, 40 may be attached to the index and/or middle finger portions 4, 5 up to a point substantially corresponding to the proximal interphalangeal joints, and no further. In this way, the relative movements of the intermediate and distal phalanges may be preserved, whilst still providing support for splaying tissues surrounding an incision.

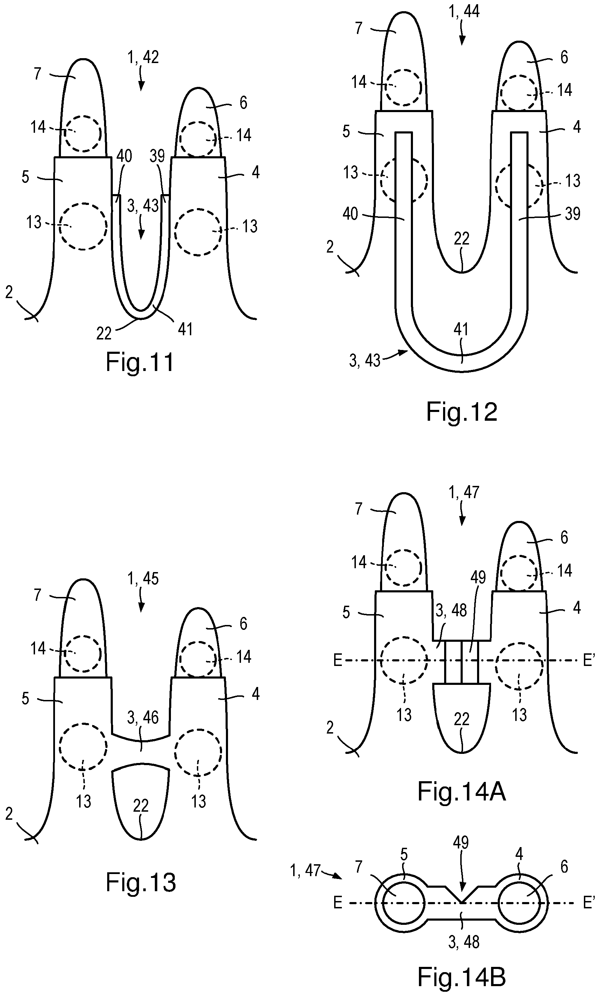

Referring also to Figure 11, a portion of a ninth tissue splayer 42 useful for understanding the present invention and including the index and middle finger portions 4, 5 is shown.

The ninth tissue splayer 42 is the same as the eighth tissue splayer 37, except that the torsion spring 38 is replaced by a flat spring 43. The flat spring 43 is shaped to fit between the index and middle finger portions 4, 5, and in a neutral (unstressed) configuration corresponds to the predetermined angular separation *θₒ*. The flat spring 43 is shaped so as to form the first portion 39, second portion 40 and spring portion 41 (which experiences the majority of applied stress) as different portions of the single flat spring 43.

In some examples, the flat spring 43 may be replaced by other types of spring such as, for example, a wire spring (not shown), a leaf spring (not shown), or any other suitable type of spring. In other examples, the first and second portions 39, 40 need not be formed as a single piece with the spring portion 41. Instead, the first and second portions 39, 40 may be separate elements which are attached to a separate spring portion 41 in the form of, for example, a flat spring, a wire spring, a leaf spring, a helical spring, a torsion spring, a constant force spring, or any other suitable type of spring.

Referring also to Figure 12, a portion of a tenth tissue splayer 44 useful for understanding the present invention and including the index and middle finger portions 4, 5 is shown.

The tenth tissue splayer 44 is the same as the ninth tissue splayer, except for the placement of the splay mechanism 3 in the form of a flat spring 43.

Instead of being located between the index and middle finger portions 4, 5, in the tenth tissue splayer 44 the flat spring 43 is disposed to overlie the dorsal surfaces of the user's index and middle fingers 6, 7 in use. The first and second portions 39, 40 may extend to a spring portion 41 disposed over the dorsal surface of a palm portion of the glove 2.

The index and middle finger portions 4, 5 are attached to splay mechanism 3 in the form of a flat spring 38 as described hereinbefore. If the attachment extends only up to the proximal interphalangeal joints 13, then the intermediate and distal phalanges of a user are free to curl away from the flat spring 43, whilst still being supported for a splaying motion. Even if the index and middle finger portions 4, 5 are attached to the splay mechanism 3 beyond the proximal interphalangeal joints 13, user dexterity and dynamic control may be preserved by forming the index and middle finger portions 4, 5 from an elastomeric material. In this way, the user may deform the index and/or middle finger portions 4, 5 away from the splay mechanism whilst applying a force.

In some examples, the flat spring 43 may be replaced by other types of spring such as, for example, a leaf spring (not shown). In other examples, the first and second portions 39, 40 need not be formed as a single piece with the spring portion 41. Instead, the first and second portions 39, 40 may be separate elements which are attached to a separate spring portion 41 in the form of, for example, a flat spring, a wire spring, a leaf spring, a helical spring, a torsion spring, a constant force spring, or any other suitable type of spring.

In other examples, the splay mechanism 3 in the form of a flat spring 43 (or other suitable type of spring) may be disposed overlying ventral surfaces of a user's fingers and/or palm. A ventral configuration should function identically in relation to supporting a splaying motion of the index and middle fingers 6, 7. However, in may be more difficult to realise some of the hereinbefore described advantages for the dexterity of a user. For example, compared to a dorsal placement of the flat spring 43, in a ventral configuration, depending on the length of first and second portions 39, 40, the flat spring 43 may interfere with curling of the index and/or middle fingers 6, 7.

Referring also to Figure 13, an eleventh tissue splayer 45 useful for understanding the present invention is shown.

The eleventh tissue splayer 45 is the same as the first or fifth tissue splayers 20, 32, except that instead of a spacer 21, the eleventh tissue splayer 45 includes a splay mechanism 3 in the form of a necked spacer 46. The necked spacer 46 connects between the index finger portion 4 and the middle finger portion 5. Using a necked spacer 46 may permit fine-tuning of the amount of biasing force *F* provided by the splay mechanism 3.

In other examples, instead of a concave, necked spacer 46, the splay mechanism 3 may take the form of a convex, bulged spacer (not shown). In other words, the splay mechanism 3 may take the form of a spacer which has minimum cross-sectional area at the points of connection to the index and middle finger portions 4, 5, and maximum cross-section at a point between the index and middle finger portions 4, 5. Such a convex spacer may permit reducing the biasing force *F* (compared to a spacer 21 of substantially uniform thickness), whilst minimising the possibility of buckling when compressed.

Referring also to Figure 14A, a twelfth tissue splayer 47 useful for understanding the present invention is shown. Referring also to Figure 14B, a cross-section is shown corresponding to the line E-E' shown in Figure 14A.

The twelfth tissue splayer 47 is the same as the first or fifth tissue splayers 20, 32, except that instead of a spacer 21, the twelfth tissue splayer 47 includes a splay mechanism 3 in the form of a spacer 48 which includes a cut-away portion 49. The cut-away portion 49 may take any suitable shape, for example, the cut-away portion may be v-shaped as shown in Figures 14A and 14B. The cut-away portion 49 is not limited to a v-shape, and may be curved. The cut-away portion 49 may serve several purposes. For example, the cut-away portion 49 may be shaped and or arranged so as to improve visibility between the index and middle finger portions 4, 5, when the user views an incision being splayed with their eye(s) positioned over the dorsal portion of the glove 2.

Improved visibility may correspond to a reduced projected area of the spacer 48 when viewed from over the dorsal portion of the glove 2. The projected area is reduced by comparison with a spacer, for example spacer 21, which is the same except as the spacer 48 except for the absence of a cut-away portion 49.

Additionally and/or alternatively to improving visibility of tissues being splayed (or spread), a cut-away portion 49 may also be used to fine-tune the mechanical response and biasing force *F* provided by the spacer 48.

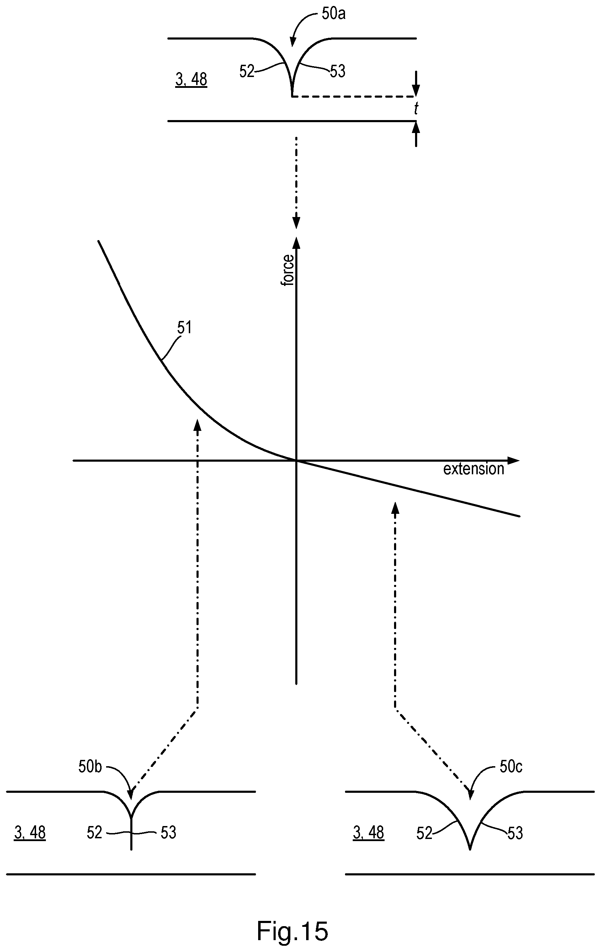

For example, referring also to Figure 15, a profiled cut-away portion 50 is illustrated in a neutral configuration 50a, in a compressed configuration 50b, and in a tensioned configuration 50c.

A schematic force-extension curve 51 corresponding to the spacer 48 having a profiled cut-away portion 50 is also shown. The profiled cut-away 50 has first and second opposed faces 52, 53, and extends through the spacer 48 to leave a residual thickness *t* connecting the portions of the spacer 48 to either side of the profiled cut-away 50.

When the spacer 48 is compressed, parts of the opposed faces 52, 53 are brought into contact, as illustrated by the compressed configuration. The effective minimum cross-sectional area of the spacer 48 is increased, which consequently increases the biasing force *F* which must be overcome in order to further compress the spacer 48. By contrast, when the spacer 48 is tensioned, the opposed faces 52, 53 are pulled further apart, and the biasing force *F* may be dominated by the narrowest region, with thickness *t.*

In this way, by forming a spacer 48 having a profiled cut-away portion 50, and by control of the shapes of faces 52, 53, the spacer 48 may be provided with asymmetric and/or non-linear (non-Hookean) force-extension behaviour. For example, as illustrated in Figure 15, the biasing force *F* provided in tension when the angular separation is *θ* > *θₒ* may be lower than the biasing force F provided in tension when the angular separation is *θ* > *θₒ*. This may provide improved dexterity for the user, whilst still providing support for a splaying motion.

Although the spacer 48 has been illustrated with a single cut-away portion 49, 50, in other examples a spacer (not shown) may include two or more cut-away portions 49, 50.

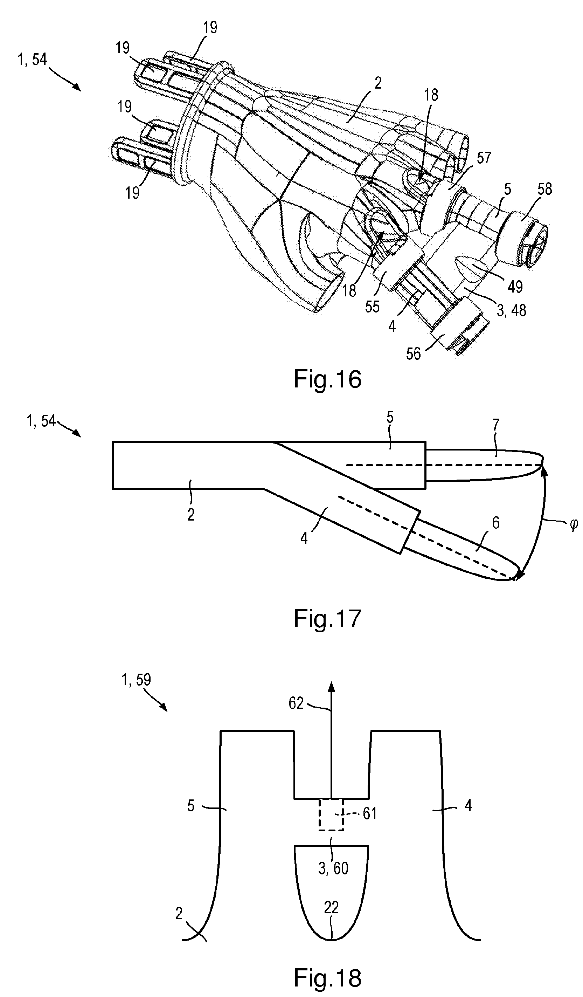

Referring also to Figures 16 and 17, a thirteenth tissue splayer 54 useful for understanding the present invention is shown.

Tissue splayers 1, 20, 23, 25 and so forth have been described in which the index and middle finger portions 4, 5 are separated by a predetermined angular separation *θₒ* when in a neutral (un-stressed) configuration. In the neutral configuration, the tissue splayer 1 is not subjected to external forces. In other words, the tissue splayer 1 is subject only to internal stresses, for example residual stresses resulting from fabrication, and so forth.

In the examples described hereinbefore, the predetermined angular separation *θₒ* has been illustrated as being substantially co-planer with a palm (ventral) portion of the glove 2. However, the predetermined angular separation *θₒ* is not restricted to this plane. For example, the predetermined angular separation *θₒ* of the thirteenth tissue splayer 54 has a component *φ* which lies out of, and substantially perpendicular to, a plane substantially parallel with a palm (ventral) portion of the glove 2.

Having the angle of splaying rotated with respect to the hand of the user may be more comfortable and may place less strain on the user's hand. For example, to obtain a desired separation between the tips of index and middle fingers 6, 7 may require a significant splaying (abduction) of the fingers 6, 7, which may place strain on the joints and/or muscles. By contrast, obtaining the same separation with a combination of abduction, flexion and/or extension by, i.e. by rotating the angle of splaying, the total abduction may be relatively reduced.

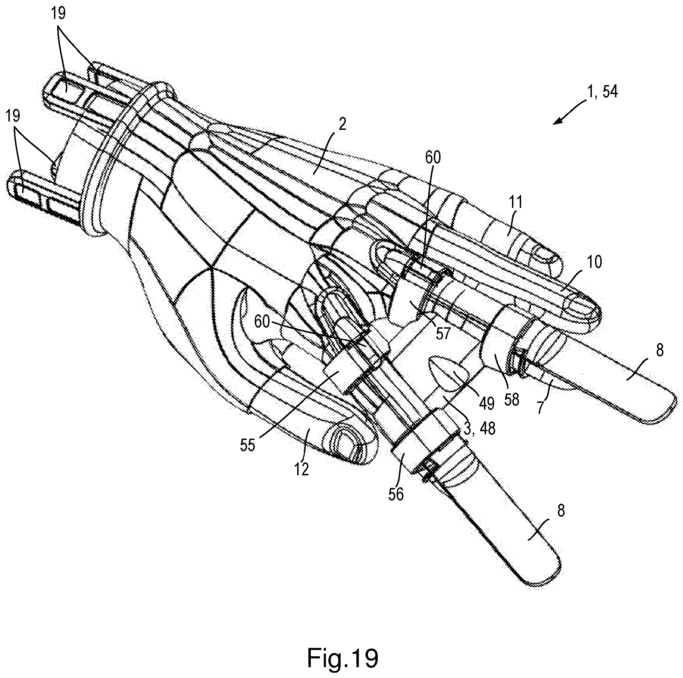

The thirteenth tissue splayer 54 also includes first and second reinforced regions 55, 56 attached to or integrally formed with the index finger portion 4, and third and fourth reinforced regions 57, 58 attached to or integrally formed with the middle finger portion 5. The reinforced regions 55, 56, 57, 58 may be used for receiving and/or attaching one or more finger-extension members 8 (see also Figures 19 to 28). The thirteenth tissue splayer 54 includes a splay mechanism 3 in the form of a spacer 48 which includes a cut-away portion 49.

Referring also to Figure 18, a fourteenth tissue splayer 59 useful for understanding the present invention is shown.

The fourteenth tissue splayer 59 is the same as the first tissue splayer 20, except that the splay mechanism 3 in the form of a spacer 60 includes a light source 61 configured to emit light 62 directed away from the connected point 22 at which the index and middle finger portions 4, 5 are joined together. In other words, the light source 61 may be arranged so as to illuminate tissues being splayed apart using the index and middle fingers 6, 7 of a user (or corresponding finger-extension members 8), supported by the tissue splayer 59.

The spacer 60 may be molded around the light source 61. Alternatively, the spacer 60 may be formed including a void configured to receive the light source 61.

Any of the spacers 21, 24, 26, 29, 28, 34, 46, 48 of the first to sixth tissue splayers 20, 23, 25, 27, 32, 33, eleventh tissue splayer 45 or twelfth tissue splayer 47 may be adapted to include or receive a light source 61.

Referring also to Figure 19, the thirteenth tissue splayer 54 is illustrated with a finger-extension member 8 attached to each of the index finger portion 4 and the middle finger portion 5. In the illustration of Figure 19, the finger-extension members 8 have been attached to the index and middle finger portions 4, 5 using clips 60 integrated with the finger-extension members 8 and the first to fourth reinforced regions 55, 56, 57, 58.

In general, the finger-extension members 8 may be attached to the index and/or middle finger portions 4, 5 using any suitable means, including but not limited to those described hereinafter with reference to Figures 20A to 28D.

Referring also to Figure 20A, a first configuration 61 of an index finger portion 4 for reception of a finger-extension member 8 is shown. Referring also to Figure 20B, a cross-section is shown along the line labelled F-F' in Figure 20A.

The first configuration 61 is illustrated and described in relation to the index finger portion 4 as an example. However, the first configuration 61, modified as necessary, is equally applicable to the middle finger portion 5 and the third and fourth reinforced regions 57, 58.

The first configuration 61 includes the first reinforced region 55 and the second reinforced region 56 which are attached to, or integrated with, the index finger portion 4. Each of the reinforced regions 55, 56 takes the form of an annular region around the index finger portion 4. The first and second reinforced regions 56, 57 may include, or take the form of, regions in which a wall thickness of the glove 2 is increased relative to the remainder of the index finger portion 4. When the first and second reinforced regions 56, 57 are formed as portions of the index finger portion 4, the wall thickness may be increased inwards and/or outwards (with respect to the void for receiving the index finger 6) of the surrounding portions of the index finger portion 4. Thickening the wall inwards may provide a tighter fit around the index finger 6, which may provide pre-stress for stabilising one or more finger-extension members 8 coupled to the reinforced regions 56, 57. Alternatively, the first and second reinforced regions 56, 57 may be formed separately from the glove 2 and subsequently attached or connected to the index finger portion 4.

The first reinforced region 55 may be arranged so as to lie substantially over the proximal phalanx of a user's index finger 6 when the glove 2 is worn. The second reinforced region 56 may be arranged so as to lie substantially over the intermediate phalanx of a user's index finger 6 when the glove 2 is worn. In other words, the first and second reinforced regions 55, 56 may be arranged to avoid the joints 13, 14 of a user's fingers when the glove 2 is worn, with the aim of preserving mobility of the index finger 6.

Each reinforced region 55, 56 includes one or more through passages 62, each of which is oriented substantially parallel with the index finger portion 4 (when in the unstressed state). The through passages 62 may be used to receive corresponding finger-extension members 8. Although shown in Figures 20A and 20B as including two through-passages arranged on opposite sides of the index finger portion 4, each of the first and second reinforced regions 55, 56 may include any number of through passages 62. Furthermore, the through passages 62 may in general be arranged at any point around the periphery of the index finger portion 4. Preferably, through passages 62 in the first reinforced region 55 should be aligned with corresponding through passages 62 in the second reinforced region 56. The through passages 62 may have a cross-sectional shape corresponding to the finger-extension members 8. For example, in Figure 20B, the through passages 62 are configured to receive finger-extension members 8 having a curved cross-section in order to better conform to the natural shape of a finger.

Referring also to Figure 21, a first example of a finger-extension member 8, 63 is shown (also referred to as the "first" finger-extension member 63).

Although illustrated and described in relation to the index finger portion 4 as an example, the same configuration, modified as necessary, is equally applicable to the middle finger portion 5 and the third and fourth reinforced regions 57, 58.

The first finger-extension member 63 extends along a longitudinal direction from a first end 64 to a second end 65, and includes a number of barbs 66 spaced along the length of the first finger-extension member 63. When received by the index finger portion 4 (or equivalently the middle finger portion 5), the longitudinal direction of the first finger-extension member 63 extends along the length of the index finger portion 4 (or equivalently the middle finger portion 5). The second end 65 is for contacting a patient in use.

The barbs 66 are provided so that the finger-extension members 63 may be readily passed through the through passages 62 in a direction from the fingertip towards the connection point 22, but not in the reverse direction.

Referring also to Figure 22, a first finger-extension member 63 received by the first and second reinforced regions 55, 56 is shown.

In this example, the barbs 66 are oriented so that the first end 64 of the first finger-extension member 63 may be inserted into a through passage of the second reinforced region 55, then pushed into position until the barb 66 closest to the first end 64 engages with the first reinforced region 55. Alternatively, the orientation of the barbs 66 could be reversed and the first finger-extension member 63 could be inserted through the first reinforced region 55 first.

Barbs 66 have been illustrated as extending away from the index finger portion 4. However, additionally or alternatively, inward facing barbs (not shown) may be included to dig into the material forming the index finger portion4 and/or reinforced regions 55, 56. Barbs 66 are not the only means for securing/attaching finger-extension members 8 to the index or middle fingers 6, 7. In other examples, finger-extension members 8 may include any alternative securing means which are configured to engage one or more reinforced portions 55, 56, 57, 58 of the index finger portion 4 or the middle finger portion 5.

For example, referring also to Figures 23 and 24, a second finger-extension member 8, 67 is shown.

The second finger-extension member 67 may be used with the first configuration 61. Although illustrated and described in relation to the index finger portion 4 as an example, the second finger-extension member 67 is equally applicable to the middle finger portion 5 and the third and fourth reinforced regions 57, 58 using the first configuration 61.

The second finger-extension member 67 is the same as the first finger-extension member 63, except that the barbs 66 are replaced with a clip 68. In the example illustrated in Figures 23 and 24, the clip 68 is arranged proximate to the first end 64 of the second finger-extension member 67. The second end 65 of the second finger-extension member 67 is inserted through a through-passage 62 of the first reinforced region 55, followed by a through-passage 62 of the second reinforced region 56. The second finger-extension member 67 is pushed further away from the connection point 22 until the clip 68 has engaged the first reinforced region 55.

In other examples the clip 68 may be placed at a different position between the first and second ends 64, 65. In further examples, the second finger-extension member 67 may include two or more clips 68.

The first configuration 61 has been described as including first and second reinforced regions 55, 56 attached to or formed as part of the index finger portion 4. The middle finger portion 5 also includes a pair of reinforced regions 57, 58. However, the configuration of index and/or middle finger portions 4, 5 to receive finger-extension members 8 is not limited to a pair of reinforced regions 55, 56, 57, 58 for each of the index and middle finger portions 4, 5. In other examples, each finger portion 4, 5 may include a single reinforced region, or each finger portion may include three or more reinforced regions.

In the first configuration 61, finger-extension members 8, 63, 67 are received through reinforced portions 55, 56, 57, 58 of the index and/or middle finger portions 4, 5. However, in other examples, finger-extension members 8, 63, 67 may alternatively be received under reinforced portions 55, 56, 57, 58 of the index and/or middle finger portions 4, 5

Referring also to Figure 25A, a second configuration 69 of an index finger portion 4 for reception of a finger-extension member 8, 63, 67 is shown. Referring also to Figure 25B, a cross-section is shown along the line labelled G-G' in Figure 25A. Referring also to Figure 25C, a cross-section is shown along the line labelled H-H' in Figure 25A.

The second configuration 69 is illustrated and described in relation to the index finger portion 4 as an example. However, the second configuration 69, modified as necessary, is equally applicable to the middle finger portion 5 and the third and fourth reinforced regions 57, 58.

The second configuration 69 differs from the first configuration 61 in that the reinforced regions 55, 56, 57, 58 do not include through-passages 62. In other respects, the reinforced regions 55, 56, 57, 58 of the second configuration 69 are the same as the reinforced regions 55, 56, 57, 58 of the first configuration.

Instead of through-passages 62, the index finger portion 4 of the second configuration 69 includes a first pair of slits 70a, 70b provided on either side of the first reinforced region 55, and a second pair of slits 71a, 71b provided on either side of the second reinforced region 56. The slits 70a, 70b, 71a, 71b may be provided through the thickness of a wall defining the index finger portion 4. Identical slits 70a, 70b, 71a, 71b may also be provided on the opposite side of the index finger portion 4.

Although shown in Figures 25A and 25C as including slits 70a, 70b, 71a, 71b arranged on opposite sides of the index finger portion 4, each of the first and second reinforced regions 55, 56 may be bracketed by any number of pairs of slits 70a, 70b, 71a, 71b. Furthermore, the slits 70a, 70b, 71a, 71b may in general be arranged at any point around the periphery of the index finger portion 4. Preferably, slits 70a, 70b, 71a, 71b are arranged to lie along straight lines extending longitudinally along the length of the index finger portion 4.

Referring also to Figure 26, the reception of a first finger-extension member 63 by an index finger portion 4 according to the second configuration 69 is shown in a cross-section view.

The first finger-extension member 63 is threaded through the first and second pairs of slits 70a, 70b, 71a, 71b, passing beneath the first and second reinforced regions 55, 56. The barbs 66 of the first finger-extension member 63 engage with the material of the index finger portion 4 around the slits 70a, 70b, 71a, 71b to secure the first finger-extension member 63. Voids 72 may be formed between the first finger-extension member 63 and the user's gloved or un-gloved index finger 6, depending on the thickness and elasticity of the material used to form the index finger portion 4.

Referring also to Figure 27, the reception of a second finger-extension member 67 by an index finger portion 4 according to the second configuration 69 is shown in a cross-section view.

The second finger-extension member 67 is threaded through the first and second pairs of slits 70a, 70b, 71a, 71b, passing beneath the first and second reinforced regions 55, 56. The clip 68 of the second finger-extension member 67 engages with the material of the first reinforced region 55 to secure the second finger-extension member 67. Voids 72 may be formed between the second finger-extension member 67 and the user's gloved or un-gloved index finger 6, depending on the thickness and elasticity of the material used to form the index finger portion 4.

In an alternative example, the second slit 70b of the first pair and the first slit 71a of the second pair may be omitted. The finger extension member 8, 63, 67 may instead be passed inside the index finger portion 4 through the first slit 70a of the first pair (before the first reinforced region 55) and back out through the second slit 71b of the second pair (after the second reinforced region 56).

In the first and second configurations 61, 69, finger-extension members 8, 63, 67 have been received via through-passages 62 or slits 70a, 70b, 71a, 71b formed through the index finger portion 4 or reinforced regions 55, 56 thereof. Equivalent configurations may be applied to the middle finger portion 5 and reinforced regions 57, 58 thereof. However, through-passages, slits, holes and so forth through the finger portions 4, 5 and/or reinforced regions 55, 56, 57, 58 thereof are not essential, and other methods of connecting finger-extension members 8 may be used.

Referring also to Figure 28A, a third configuration 73 of an index finger portion 4 for reception of a third finger-extension member 8, 74 is shown. Referring also to Figure 28B, a cross-section is shown along the line labelled J-J' in Figure 28A. Referring also to Figure 28C, a cross-section is shown along the line labelled K-K' in Figure 28A. Referring also to Figure 28C, a plan view of the third finger-extension member 8, 74 is shown along a direction parallel to the lines labelled J-J' and K-K' in Figure 28A.

The third finger-extension member 74 includes a number of clips 75a, 75b, 75c, 75d. Each clip 75a, 75b, 75c, 75d is configured to clip around and secure the index finger portion 4. A first pair of the clips 75a, 75b is spaced apart along the length of the third finger-extension member 74 so as to bracket the first reinforced region 55 when the third finger-extension member 74 is secured (in this case clipped) to the index finger portion 4. Similarly, a second pair of the clips 75c, 75d is positioned along the length of the third finger-extension member 74 so as to bracket the second reinforced region 56 when the third finger-extension member 74 is secured to the index finger portion 4. In this way, the clips 75a, 75b, 75c, 75d secure the third finger-extension member 74 to the index finger portion 4, whilst the reinforced regions 55, 56 prevent slipping of the third finger-extension member 74 parallel to the longitudinal (length) direction of the index finger portion 4.

The third configuration 73 and the third finger-extension member 74 have been illustrated and described in relation to the index finger portion 4 as an example. However, the third configuration 73 and the third finger-extension member 74, modified as necessary, are equally applicable to the middle finger portion 5 and the third and fourth reinforced regions 57, 58.

### Modifications

It will be appreciated that many modifications may be made to the examples hereinbefore described. Such modifications may involve equivalent and other features which are already known in the design and use of surgical splayers and/or retractors, and which may be used instead of, or in addition to, features already described herein. Features of one example may be replaced or supplemented by features of another example.

Although finger extension members 8, 63, 67, 74 have been illustrated as being substantially straight between first and second ends 64, 65, this is not essential. In some examples, finger extension members 8, 63, 67, 74 may be curved between the first and second ends 64, 65.

The glove 2 has been illustrated as omitting a ring finger portion (not shown), a little finger portion (not shown), and a thumb portion (not shown). However, in other examples, the glove 2 may include one or more of a ring finger portion (not shown), a little finger portion (not shown) and/or a thumb portion (not shown). When included, a ring finger portion (not shown), a little finger portion (not shown) and/or a thumb portion (not shown) may fully or partially encapsulate the respective digit of a user when worn. When included, a ring finger portion (not shown), a little finger portion (not shown) and/or a thumb portion (not shown) may leave the tip of the respective digit of a user exposed when worn.

Although examples have been described in which the tissue splayer 1, 20, 23, 25, 27, 32, 33, 35, 37, 42, 44, 45, 47, 54, 59 includes the glove 2 (also referred to as glove portion), is it not essential for the glove 2 to cover all or over part of the user's palm. In some examples, the glove 2 may include only the index finger portion 4 and the middle finger portion 5. In other examples, the glove portion 2 may be omitted altogether and the splay mechanism 3 may be received directly over a surgical glove (not shown).

Although examples have been described in which the splay mechanism 3 provides a biasing force to urge the index finger portion 4 and middle finger portion 5 towards a predetermined angular separation, alternative mechanisms may be used which may also substantially preserve the dexterity of a user. For example, a spacer 21 or a spring 36, 38 may be replaced by a rotary or linear ratchet mechanism (geared or friction based) in order to permit the index finger 6 and middle finger 7 to be moved (splayed) apart with little or no resistance, whilst the splay mechanism resists allowing the index finger 6 and middle finger 7 to be moved back together (until a release mechanism is actuated).

For example, referring also to Figures 29 to 33, a fifteenth tissue splayer 76 is shown. The fifteenth tissue splayer is within the scope of the appended claims. Figures 29 through 32 show projections of the fifteenth tissue splayer 76 from a range of different angles, whilst Figure 33 schematically illustrates the fifteenth tissue splayer 76 with a torsion spring 77 installed. The torsion spring 77 is not shown in Figures 29 through 32.

The fifteenth tissue splayer 76 includes an index finger portion 4 in the form of a first rigid annulus 78 for receiving an index finger 6 of a user and a middle finger portion 5 in the form of a second rigid annulus 79 for receiving a middle finger 7 of a user.

A first pivot joint 80 joint couples the index finger portion 4 to the middle finger portion 5 to permit the middle finger portion 5 to rotate relative to the index finger portion 4 about a first pivot axis 81. The first pivot joint 80 is configured to sit, in use, roughly between the metacarpophalangeal joints 15 of the index 4 and middle 5 fingers, over the dorsal surface of a user's palm. The index finger portion 4 includes a first dorsal extension 82 which extends from the first rigid annulus 78 to the first pivot joint 80. Similarly, the middle finger portion 5 includes a second dorsal extension 83 extending from the second rigid annulus 79 to the first pivot joint 80. A cylindrical protrusion 84 extends upwards from the first dorsal extension 82 and is concentric with the first pivot axis 81. The cylindrical protrusion 84 may form part of the first pivot joint 80 (as illustrated in Figures 29 to 33), although this is not essential. In use, the cylindrical protrusion 84 is gripped by the torsion spring 77 to provide resistance to moving the index and middle finger portions 4, 5 closer together.

A second pivot joint 85 couples the index finger portion 4 to the middle finger portion 5 to permit the middle finger portion 5 to rotate relative to the index finger portion 4 about the first pivot axis 81. The second pivot joint 80 is configured to sit, in use, roughly between the metacarpophalangeal joints 15 of the index 4 and middle 5 fingers, below the palmar surface of a user's palm. The second pivot joint 85 is co-axial with the first pivot joint 80 and the first pivot axis 81. The index finger portion 4 includes a first palmar extension 86 which extends from the first rigid annulus 78 to the second pivot joint 85. Similarly, the middle finger portion 5 includes a second palmar extension 87 extending from the second rigid annulus 79 to second pivot joint 85.

Referring in particular to Figure 33, a first splay mechanism 88 is provided by the interactions of the torsion spring 77 with the index finger portion 4 and the middle finger portion 5. The first splay mechanism 88 is configured to permit rotation of the middle finger portion 5 away from the index finger portion 4 about the first pivot axis 81, and to resist rotation of the middle finger portion 5 towards the index finger portion 4 about the first pivot axis 81 (and vice versa). The torsion spring 77 is formed from a wire having a first end 89 and a second end 90 connected by a coil portion 91. The first end 89 of the torsion spring 77 extends tangentially from the coil portion 91 and is secured to the middle finger portion 5, for example by reception into a groove 92 provided in the second rigid annulus 79. The coil portion 91 of the torsion spring 77 is received over the cylindrical protrusion 84 of the index finger portion 4. An inner radius of the coil portion 91 is smaller than an outer radius of the cylindrical protrusion 84, such that a moment must be applied between the first and second ends 89, 90 in order to expand the coil portion 91 sufficiently to allow reception over the cylindrical protrusion 84. In this way, the torsion spring 77 is fixed to the middle finger portion 5 and tightly grips the index finger portion 4 via the cylindrical protrusion 84.

The sense of rotation of the coil portion 91 is such that a moment applied about the first pivot axis 81 to cause the index and middle finger portions 4, 5 to move (splay) apart acts to cause the coil portion 91 to open up, reducing the friction gripping the cylindrical protrusion 84. In this way, rotation of the middle finger portion 5 away from the index finger portion 4 about the first pivot axis may be caused by a user using their own muscles to splay the index and middle fingers 6, 7 apart. However, a moment applied about the first pivot axis 81 to cause the index and middle finger portions 4, 5 to move back together acts to cause the coil portion 91 to contract, increasing the grip on the cylindrical protrusion 84 and resisting any rotation of the first and second pivot joints 80, 85 in that direction. The first splay mechanism 88 is analogous to the spring locks used in some prior art roller blinds.

The grip of the coil portion 91 on the cylindrical protrusion 84 may be reduced (or relaxed) to permit moving the index and middle finger portions 4, 5 back together by actuating a release mechanism in the form of a lever 93 secured to the second end 90 of the torsion spring 77.

In this way, once a user has splayed their index and middle fingers 6, 7 to a desired width, the first splay mechanism 88 locks to provide support for the index and middle fingers 6, 7 in the desired position. Since splaying of the index and middle fingers 6, 7 is still driven by the user's own muscles without addition of any mechanical advantage, application of excessive retraction force to tissues surrounding an incision may be avoided. This also helps to preserve the dexterity of the user, since they retain precise control of, and feedback from, their index and middle fingers 6, 7. Strain on the index and middle fingers 6, 7 is avoided by the first play mechanism 88 only permitting movement in one direction.

The index finger portion 4 is configured to receive a finger-extension member 8. For example, as shown in Figure 29 to 32, a structure 94 attached to, or integrally formed as part of, the first rigid annulus 78 defines a number of passages 95 positioned at angular intervals around the circumference of the first rigid annulus 78. Any of the passages 95 may receive a finger extension member 8 substantially parallel to the index finger portion 4 (and index finger 6 in use), allowing a user to configure the relative position of a finger extension member 8 for splaying a particular incision.

The middle finger portion 5 is similarly configured to receive a finger-extension member 8, including a structure 96 attached to, or integrally formed as part of, the second rigid annulus 79 and defining a number of passages 97 positioned at angular intervals around the circumference of the second rigid annulus 79.

In the example shown in Figures 29 to 32, the index and middle finger portions 4, 5 each receives a fourth finger-extension member 98. Each fourth finger-extension member 98 includes an annulus 99 defining a through-hole 100 for reception of a corresponding finger 6, 7 in use. The annulus 99 may help to stabilise the fourth finger-extension member 98 in use. Any fourth finger-extension member 98 may optionally support an electronic package 9 and/or a light source as described hereinbefore.

In the example shown in Figures 29 to 32, an end of the fourth finger-extension member 98 passes through a passage 95, 97 of the index or middle finger portion 4, 5 and is secured using barbs in a similar way to the first finger-extension member 63. However, the fourth finger-extension member 98 may alternatively be received, connected and/or retained using any method described in relation to the first, second and/or third finger-extension members 63, 67, 74.

In other examples, the roles of the index and middle finger portions 4, 5 may be reversed, so that the first end 89 is secured to the index finger portion 4 and the coil 91 grips a cylindrical protrusion of the middle finger portion 5.

Although shown in Figures 29 to 32 as receiving a pair of fourth finger-extension members 98, the index and/or middle finger portion 4, 5 of the fifteenth tissue splayer 76 may be adapted for reception or connection of any of the first to third finger-extension members 63, 67, 74 described hereinbefore.

Although described in Figures 29 to 32 as being worn on a user's left hand, the fifteenth tissue splayer 76 may be configured to be worn right-handed or may be configured to be worn on either hand (universal).

The fifteenth tissue splayer 76 is designed to be worn over a pair of surgical gloves (not shown). Similarly, the through-holes 100 of the fourth finger-extension members 98 are configured to receive a user's fingers 6, 7 whilst wearing surgical gloves (not shown).

The fifteenth tissue splayer 76 omits the glove 2, or at least any portions of the glove 2 beyond the index and middle finger portions 4, 5. This may make the fifteenth tissue splayer 76 comparatively easier to put on and/or take off. This may be advantageous, especially when a user needs to temporarily remove the splayer during a procedure.

The fifteenth tissue splayer 76 may be adapted for different sizes of user fingers 6, 7 by inserting one or more spacing rings (not shown) into the first rigid annulus, 78, the second rigid annulus 79 and/or the annulus 99 of any fourth finger-extension members 98. Alternatively, adjustable mechanisms may be included for altering internal radii of the first rigid annulus, 78, the second rigid annulus 79 and/or the annulus 99 of any fourth finger-extension members 98.

The fifteenth tissue splayer 76 has been described including a first splay mechanism 88 utilising a friction ratchet provided by a torsion spring 77. However, alternative mechanisms may be used to provide uni-directional movement between the index and middle fingers portions 4, 5. For example, the first splay mechanism 88 may be replaced with a mechanical ratchet including a gear (not shown) and a pawl (not shown). If the precision afforded by a single gear and pawl combination is not sufficient for an application, a splay mechanism may use a gear in combination with two or more pawls, with the pawls configured to sequentially engage the gear at increments of rotation angle less than an angular separate the gear teeth. In other examples a splay mechanism may include two or more concentric gears and corresponding pawls, with each concentric gear offset from each other concentric gear by a small rotation. For example the first pivot joint 80 may include a first gear and pawl ratchet whilst the second pivot joint 85 includes a second gear and pawl ratchet offset from the first by half the angular separation of the gear teeth. In general, the first splay mechanism 88 may be replaced by any mechanism providing substantially free movement of the middle finger portion 5 away from the index finger portion 4 (or vice versa), whilst resisting or preventing movement of the middle finger portion 5 towards the index finger portion 4 (or vice versa).

Any splay mechanism used may preferably include a release mechanism configured such that actuation of the release mechanism reduces or removes the resistance of the splay mechanism to rotation of the middle finger portion 5 towards the index finger portion 4 about the first pivot axis 81. Although shown in Figure 33 as a lever 93, a release mechanism may be actuated in any suitable way, for example using a button instead of a lever.

A release mechanism is preferably arranged to permit actuation using the thumb of the same hand wearing the fifteenth tissue splayer 76. However, in some examples a release mechanism may need to be operated using the other hand.

Although shown with a pair of pivot joints 80, 85, which may improve stability, in practice only the first pivot joint 80 forming part of the first splay mechanism 88 is necessary. Although shown and described as arranged to be positioned in use over the dorsal surface of a user's palm, the first pivot joint 80 and first splay mechanism 88 may alternatively be positioned below a palmar surface of a user's palm in use.

Although the fifteenth tissue splayer 76 does not include a spacer, the fifteenth tissue splayer 76 may nonetheless include a light source 61 arranged to illuminate a splayed incision. For example, a light source 61 may be attached to either one of the index finger portion 4 or the middle finger portion 5. In some examples, a pair of light sources 61 may be used, with one attached to each of the index finger portion 4 and the middle finger portion 5. In further examples, either or both of the first and/or second pivot joints 80, 85 may support or include a light source 61 arranged to illuminate a splayed incision.

The fifteenth tissue splayer 76 is configured for splaying the index and middle fingers 6, 7 in a single plane, substantially parallel to the plane of the user's palm. However, in other examples a third pivot joint 101 (Figure 34) and a second splay mechanism 102 (Figure 34) may be mechanically coupled in series with the first pivot joint 80 and first splay mechanism 88.

Referring also to Figure 34, a sixteenth tissue splayer 103 is schematically illustrated. The sixteenth tissue splayer is within the scope of the appended claims.

A middle finger portion 5 is coupled to a first (or intermediate) segment 104 of an index finger portion 4 by a first pivot joint 80 and a first splay mechanism 88, to provide unidirectional rotation (see arrow in Figure 34) about a first pivot axis 81 as explained hereinbefore in relation to the fifteenth tissue splayer 76. Additionally, the first segment 104 of the index finger portion 4 is coupled to a second (or main) segment 105 of the index finger portion 4 by a third pivot joint 101 and a second splay mechanism 102 to permit unidirectional rotation about a second pivot axis 106. The second pivot axis 106 is at an angle to the first pivot axis 81. In the example shown in Figure 34 the second pivot axis 106 is perpendicular to the first pivot axis 81, but other angles may be used and indeed may be more ergonomic. The third pivot joint 101 and a second splay mechanism 102 enable the second segment 105 of the index finger portion 4 to be rotated relatively freely away from the plane of rotation of the first pivot joint 80, whilst resisting rotation in the opposite sense. The third pivot joint 101 should be positioned so that, in use, the user's index finger 6 may flex substantially freely about the metacarpophalangeal joint (at least within a range of motion). The third pivot joint 101 may be of any suitable type, for example the same as, or similar to, the first pivot joint 80. The second splay mechanism 102 may be the same as, or similar to, the first splay mechanism 88. The second splay mechanism 102 may include a release mechanism the same as, or similar to, the first splay mechanism 88.

In this way, a user may be able to splay tissues using a movement having a component which lies out of, and substantially perpendicular to, a plane substantially perpendicular to the first pivot axis 81. Having the angle of splaying rotated with respect to the hand of the user may be more comfortable and may place less strain on the user's hand. For example, to obtain a desired separation between the tips of index and middle fingers 6, 7 may require a significant splaying (abduction) of the fingers 6, 7, which may place strain on the joints and/or muscles, even with support from a splay mechanism 88. By contrast, obtaining the same separation with a combination of abduction, flexion and/or extension by, i.e. by rotating the angle of splaying, the total abduction may be relatively reduced. Once the fingers 6, 7 are positioned as desired, the first and second splay mechanisms 88, 102 of the sixteenth tissue splayer 103 support the fingers 6, 7 in place.

The fifteenth and sixteenth tissue splayers 76, 103 include an index finger portion 4 and middle finger portion 5 which are not attached to any glove 2. However, the fifteenth and/or sixteenth tissue splayers 76, 103 could be modified so that the index and middle finger portions 4, 5 were attached to, or integrally formed with, a glove 2. The glove 2 may be configured in any way described hereinbefore.

## Claims

1. A tissue splayer (1, 20, 76) comprising:
an index finger portion (4) comprising a rigid annulus (78) for receiving an index finger (6) of a user;
a middle finger portion (5) comprising a rigid annulus (79) for receiving a middle finger (7) of a user;
a first pivot joint (80) coupling the index finger portion to the middle finger portion to permit the middle finger portion to rotate relative to the index finger portion about a first pivot axis (81), the first pivot joint configured to be located, in use, above a dorsal surface of a user's palm;
a second pivot joint (85) coupling the index finger portion (4) to the middle finger portion (5) to permit the middle finger portion to rotate relative to the index finger portion about the first pivot axis (81), the second pivot joint configured to be located, in use, below a palmar surface of a user's palm; and
a first splay mechanism (88) configured to permit rotation of the middle finger portion away from the index finger portion about the first pivot axis, and to resist rotation of the middle finger portion towards the index finger portion about the first pivot axis.

2. A tissue splayer (1, 20, 76) according to claim 1, wherein the splay mechanism (88) further comprises a release mechanism configured such that actuation of the release mechanism reduces or removes the resistance of the first splay mechanism to rotation of the middle finger portion (5) towards the index finger portion (4) about the first pivot axis (81).

3. A tissue splayer (1, 20, 76) according to claims 1 or 2, further comprising a first finger-extension member (8) connected to, received by, or integrally formed with the index finger portion (4) and a second finger-extension member (8) connected to, received by, or integrally formed with the middle finger portion (5).

4. A tissue splayer (1, 20, 76) according to claim 3, wherein the first finger-extension member (8) comprises an annulus (99) for receiving an index finger (6) of a user.

5. A tissue splayer (1, 20, 76) according to claims 3 or 4, wherein the second finger-extension member (8) comprises a rigid annulus (99) for receiving a middle finger (7) of a user.

6. A tissue splayer (1, 20, 76) according to any one of claims 1 to 5, wherein the splay mechanism (88) comprises a friction ratchet comprising a torsion spring (77).

7. A tissue splayer (1, 20, 76) according to any one of claims 1 to 5, wherein the splay mechanism (88) comprises a ratchet comprising a gear and a pawl.

8. A tissue splayer (1, 20, 76) according to any one of claims 1 to 7, further comprising:
a third pivot joint (101) configured to permit rotation about a second pivot axis which is angled relative to the first pivot axis (106); and
a second splay mechanism (102);
wherein the third pivot joint is connected in series with the first pivot joint (80) to couple the index finger portion (4) to the middle finger portion (5);
wherein the second splay mechanism is configured to permit rotation of the index finger portion away from the middle finger portion about the second pivot axis and to resist rotation of the index finger portion towards the middle finger portion about the second pivot axis.

9. A tissue splayer (1, 20, 76) according to any one of claims 1 to 8, wherein the tissue splayer is configured to be worn over a surgical glove (2).

10. A tissue splayer (1, 20, 76) according to any one of claims 1 to 9, wherein the index finger portion (4) and/or the middle finger portion (5) are attached to or integrated with a glove (2).

11. A tissue splayer (1, 20, 76) according to any one of claims 1 to 10, wherein the index finger portion (4) is configured to receive a finger-extension member (8) and/or wherein the middle finger portion (5) is configured to receive a finger-extension member (8);
each finger-extension member configured for connection to, or reception by, an index finger portion (4) or a middle finger portion (5) of the tissue splayer (1, 20, 76);
wherein the finger-extension member extends along a longitudinal direction from a first end (64) to a second end (65), and wherein when received by an index or middle finger portion the longitudinal direction of the finger-extension member extends along the length of the index or middle finger portion; and
wherein, in use, the second end is for contacting a patient.

12. A kit comprising:
a tissue splayer (1, 20, 76) according to any one of claims 5 to 11; and
one or more finger-extension members (8), each finger-extension member configured for connection to, or reception by, an index finger portion (4) or a middle finger portion (5) of the tissue splayer (1, 20, 76);
wherein the finger-extension member extends along a longitudinal direction from a first end (64) to a second end (65), and wherein when received by an index or middle finger portion the longitudinal direction of the finger-extension member extends along the length of the index or middle finger portion; and
wherein, in use, the second end is for contacting a patient.

## Patentansprüche

1. Gewebespreizer (1, 20, 76), umfassend:
einen Zeigefingerabschnitt (4), der einen starren Ring (78) zum Aufnehmen eines Zeigefingers (6) eines Benutzers umfasst;
einen Mittelfingerabschnitt (5), der einen starren Ring (79) zum Aufnehmen eines Mittelfingers (7) eines Benutzers umfasst;
ein erstes Schwenkgelenk (80), das den Zeigefingerabschnitt an den Mittelfingerabschnitt koppelt, um es dem Mittelfingerabschnitt zu ermöglichen, sich bezogen auf den Zeigefingerabschnitt um eine erste Schwenkachse (81) zu drehen, wobei das erste Schwenkgelenk dazu konfiguriert ist, sich im Gebrauch über einer Handrückenoberfläche einer Handfläche eines Benutzers zu befinden;
ein zweites Schwenkgelenk (85), das den Zeigefingerabschnitt (4) an den Mittelfingerabschnitt (5) koppelt, um zu ermöglichen, dass sich der Mittelfingerabschnitt bezogen auf den Zeigefingerabschnitt um die erste Schwenkachse (81) dreht, wobei das zweite Schwenkgelenk dazu konfiguriert ist, sich im Gebrauch unter einer Handflächenoberfläche einer Handfläche eines Benutzers zu befinden; und
einen ersten Spreizmechanismus (88), der dazu konfiguriert ist, eine Drehung des Mittelfingerabschnitts von dem Zeigefingerabschnitt weg um die erste Schwenkachse zu ermöglichen und einer Drehung des Mittelfingerabschnitts in Richtung des Zeigefingerabschnitts um die erste Schwenkachse zu widerstehen.

2. Gewebespreizer (1, 20, 76) nach Anspruch 1, wobei der Spreizmechanismus (88) ferner einen Freigabemechanismus umfasst, der derart konfiguriert ist, dass eine Betätigung des Freigabemechanismus den Widerstand des ersten Spreizmechanismus gegen eine Drehung des Mittelfingerabschnitts (5) in Richtung des Zeigefingerabschnitts (4) um die erste Schwenkachse (81) reduziert oder entfernt.

3. Gewebespreizer (1, 20, 76) nach Anspruch 1 oder 2, ferner umfassend ein erstes Fingerverlängerungselement (8), das mit dem Zeigefingerabschnitt (4) verbunden ist, durch diesen aufgenommen wird oder einstückig mit diesem gebildet ist, und ein zweites Fingerverlängerungselement (8), das mit dem Mittelfingerabschnitt (5) verbunden ist, durch diesen aufgenommen wird oder einstückig mit diesem gebildet ist.

4. Gewebespreizer (1, 20, 76) nach Anspruch 3, wobei das erste Fingerverlängerungselement (8) einen Ring (99) zum Aufnehmen eines Zeigefingers (6) eines Benutzers umfasst.

5. Gewebespreizer (1, 20, 76) nach Anspruch 3 oder 4, wobei das zweite Fingerverlängerungselement (8) einen starren Ring (99) zum Aufnehmen eines Mittelfingers (7) eines Benutzers umfasst.

6. Gewebespreizer (1, 20, 76) nach einem der Ansprüche 1 bis 5, wobei der Spreizmechanismus (88) eine Reibungsratsche umfasst, die eine Torsionsfeder (77) umfasst.

7. Gewebespreizer (1, 20, 76) nach einem der Ansprüche 1 bis 5, wobei der Spreizmechanismus (88) eine Ratsche umfasst, die ein Zahnrad und eine Sperrklinke umfasst.

8. Gewebespreizer (1, 20, 76) nach einem der Ansprüche 1 bis 7, ferner umfassend:
ein drittes Schwenkgelenk (101), das dazu konfiguriert ist, eine Drehung um eine zweite Schwenkachse zu ermöglichen, die bezogen auf die erste Schwenkachse (106) abgewinkelt ist; und
einen zweiten Spreizmechanismus (102);
wobei das dritte Schwenkgelenk in Reihe mit dem ersten Schwenkgelenk (80) verbunden ist, um den Zeigefingerabschnitt (4) an den Mittelfingerabschnitt (5) zu koppeln;
wobei der zweite Spreizmechanismus dazu konfiguriert ist, eine Drehung des Zeigefingerabschnitts von dem Mittelfingerabschnitt weg um die zweite Schwenkachse zu ermöglichen und einer Drehung des Zeigefingerabschnitts in Richtung des Mittelfingerabschnitts um die zweite Schwenkachse zu widerstehen.

9. Gewebespreizer (1, 20, 76) nach einem der Ansprüche 1 bis 8, wobei der Gewebespreizer dazu konfiguriert ist, über einem Operationshandschuh (2) getragen zu werden.

10. Gewebespreizer (1, 20, 76) nach einem der Ansprüche 1 bis 9, wobei der Zeigefingerabschnitt (4) und/oder der Mittelfingerabschnitt (5) an einem Handschuh (2) befestigt oder in diesen integriert sind/ist.

11. Gewebespreizer (1, 20, 76) nach einem der Ansprüche 1 bis 10, wobei der Zeigefingerabschnitt (4) dazu konfiguriert ist, ein Fingerverlängerungselement (8) aufzunehmen, und/oder wobei der Mittelfingerabschnitt (5) dazu konfiguriert ist, ein Fingerverlängerungselement (8) aufzunehmen;
wobei jedes Fingerverlängerungselement zur Verbindung mit einem Zeigefingerabschnitt (4) oder einem Mittelfingerabschnitt (5) des Gewebespreizer (1, 20, 76) oder zur Aufnahme durch diesen konfiguriert ist;
wobei sich das Fingerverlängerungselement entlang einer Längsrichtung von einem ersten Ende (64) zu einem zweiten Ende (65) erstreckt und wobei sich, wenn es durch einen Zeige- oder Mittelfingerabschnitt aufgenommen wird, die Längsrichtung des Fingerverlängerungselements entlang der Länge des Zeige- oder Mittelfingerabschnitts erstreckt; und
wobei im Gebrauch das zweite Ende zum Berühren eines Patienten dient.

12. Kit, umfassend:
Gewebespreizer (1, 20, 76) nach einem der Ansprüche 5 bis 11; und
ein oder mehrere Fingerverlängerungselemente (8), wobei jedes Fingerverlängerungselement zur Verbindung mit einem Zeigefingerabschnitt (4) oder einem Mittelfingerabschnitt (5) des Gewebespreizer (1, 20, 76) oder zur Aufnahme durch diesen konfiguriert ist;
wobei sich das Fingerverlängerungselement entlang einer Längsrichtung von einem ersten Ende (64) zu einem zweiten Ende (65) erstreckt und wobei sich, wenn es durch einen Zeige- oder Mittelfingerabschnitt aufgenommen wird, die Längsrichtung des Fingerverlängerungselements entlang der Länge des Zeige- oder Mittelfingerabschnitts erstreckt; und
wobei im Gebrauch das zweite Ende zum Berühren eines Patienten dient.

## Revendications

1. Écarteur de tissu (1, 20, 76) comprenant :
une partie index (4) comprenant un anneau rigide (78) destiné à recevoir un index (6) d'un utilisateur ;
une partie majeur (5) comprenant un anneau rigide (79) destiné à recevoir un majeur (7) d'un utilisateur ;
un premier joint de pivot (80) couplant la partie index à la partie majeur pour permettre à la partie majeur de tourner par rapport à la partie index autour d'un premier axe de pivot (81), le premier joint de pivot étant conçu pour être situé, lors de l'utilisation, au-dessus d'une surface dorsale d'une paume d'un utilisateur ;
un deuxième joint de pivot (85) couplant la partie index (4) à la partie majeur (5) pour permettre à la partie majeur de tourner par rapport à la partie index autour du premier axe de pivot (81), le deuxième joint de pivot étant conçu pour être situé, lors de l'utilisation, sous une surface palmaire de la paume d'un utilisateur ; et
un premier mécanisme d'écartement (88) conçu pour permettre la rotation de la partie majeur loin de la partie index autour du premier axe de pivot, et pour résister à la rotation de la partie majeur vers la partie index autour du premier axe de pivot.

2. Écarteur de tissu (1, 20, 76) selon la revendication 1, ledit mécanisme d'écartement (88) comprenant en outre un mécanisme de libération conçu de sorte que l'actionnement du mécanisme de libération réduit ou supprime la résistance du premier mécanisme d'écartement à la rotation de la partie majeur (5) vers la partie index (4) autour du premier axe de pivot (81).

3. Écarteur de tissu (1, 20, 76) selon la revendication 1 ou 2, comprenant en outre un premier élément d'extension de doigt (8) relié à la partie index (4), reçu par celle-ci, ou formé d'un seul tenant avec celle-ci et un second élément d'extension de doigt (8) relié à la partie majeur (5), reçu par celle-ci, ou formé d'un seul tenant avec celle-ci.

4. Écarteur de tissu (1, 20, 76) selon la revendication 3, ledit premier élément d'extension de doigt (8) comprenant un anneau (99) destiné à recevoir un index (6) d'un utilisateur.

5. Écarteur de tissu (1, 20, 76) selon la revendication 3 ou 4, ledit second élément d'extension de doigt (8) comprenant un anneau rigide (99) destiné à recevoir un majeur (7) d'un utilisateur.

6. Écarteur de tissu (1, 20, 76) selon l'une quelconque des revendications 1 à 5, ledit mécanisme d'écartement (88) comprenant un encliquetage à friction comprenant un ressort de torsion (77).

7. Écarteur de tissu (1, 20, 76) selon l'une quelconque des revendications 1 à 5, ledit mécanisme d'écartement (88) comprenant un encliquetage comprenant une roue dentée et un cliquet.

8. Écarteur de tissu (1, 20, 76) selon l'une quelconque des revendications 1 à 7, comprenant en outre :
un troisième joint de pivot (101) conçu pour permettre la rotation autour d'un second axe de pivot qui est incliné par rapport au premier axe de pivot (106) ; et
un second mécanisme d'écartement (102) ;
ledit troisième joint de pivot étant relié en série avec le premier joint de pivot (80) pour coupler la partie index (4) à la partie majeur (5) ;
ledit second mécanisme d'écartement étant conçu pour permettre la rotation de la partie index loin de la partie majeur autour du second axe de pivot et pour résister à la rotation de la partie index vers la partie majeur autour du second axe de pivot.

9. Écarteur de tissu (1, 20, 76) selon l'une quelconque des revendications 1 à 8, ledit écarteur de tissu étant conçu pour être porté sur un gant chirurgical (2).

10. Écarteur de tissu (1, 20, 76) selon l'une quelconque des revendications 1 à 9, ladite partie index (4) et/ou ladite partie majeur (5) étant fixées ou intégrées à un gant (2).

11. Écarteur de tissu (1, 20, 76) selon l'une quelconque des revendications 1 à 10, ladite partie index (4) étant conçue pour recevoir un élément d'extension de doigt (8) et/ou ladite partie majeur (5) étant conçue pour recevoir un élément d'extension de doigt (8) ;
chaque élément d'extension de doigt étant conçue pour un raccordement à une partie index (4) ou une partie majeur (5) de l'écarteur de tissu (1, 20, 76) ou une réception par celles-ci ;
ledit élément d'extension de doigt s'étendant le long d'une direction longitudinale à partir d'une première extrémité (64) jusqu'à une seconde extrémité (65), et lorsqu'il est reçu par une partie index ou majeur, ladite direction longitudinale de l'élément d'extension de doigt s'étendant le long de la longueur de la partie index ou majeur ; et
lors de l'utilisation, ladite seconde extrémité étant destinée à entrer en contact avec un patient.

12. Kit comprenant :
un écarteur de tissu (1, 20, 76) selon l'une quelconque des revendications 5 à 11 ; et
un ou plusieurs éléments d'extension de doigt (8), chaque élément d'extension de doigt étant conçue pour un raccordement à une partie index (4) ou une partie majeur (5) de l'écarteur de tissu (1, 20, 76) ou une réception par celles-ci ;
ledit élément d'extension de doigt s'étendant le long d'une direction longitudinale à partir d'une première extrémité (64) jusqu'à une seconde extrémité (65), et lorsqu'il est reçu par une partie index ou majeur, ladite direction longitudinale de l'élément d'extension de doigt s'étendant le long de la longueur de la partie index ou majeur ; et
lors de l'utilisation, ladite seconde extrémité étant destinée à entrer en contact avec un patient.
